(19) **Europäisches Patentamt**
**European Patent Office**
**Office européen des brevets**

(11) **EP 1 611 847 A1**

(12) **EUROPEAN PATENT APPLICATION**

(43) Date of publication:
**04.01.2006 Bulletin 2006/01**

(51) Int Cl.:
**A61B 5/145** (2006.01)

(21) Application number: **05253973.1**

(22) Date of filing: **27.06.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **28.06.2004 US 583764 P**

(71) Applicant: **Datex-Ohmeda, Inc.**
**Madison, WI 53707 (US)**

(72) Inventor: **Van Slyke, Braddon Michael**
**Arvada, CO 80004 (US)**

(74) Representative: **Hedley, Nicholas James Matthew et al**
**Kilburn & Strode**
**20 Red Lion Street**
**London WC1R 4PJ (GB)**

(54) **Validating pulse oximetry signals in the potential presence of artifact**

(57)    Detector signals in a pulse oximeter are analyzed to determine a quality of the signals in relation to physiological information content or artifact content. The analysis involves performing a transform on a signal to obtain frequency related information and analyzing the frequency related information to obtain a value independent of a shape and waveform of a spectrum of the time-based signal. In one implementation, the analysis involves the relative amplitude or power measures of red and infrared spectra. For example, the ratio of the amplitude of the fundamental peak in the red spectrum and the amplitude of the fundamental peak in the infrared spectrum may be tracked over time. In another implementation, the analysis involves consideration of the relative phase of the fundamental and harmonic components of a signal. In either case, signals including desired physiological information can be distinguished from artifact affected signals. Based on this analysis, signals can be validated or an appropriate processing algorithm can be selected.

FIG.17

**Description**

## FIELD OF THE INVENTION

The present invention relates generally to improving the identification and use of pulse oximetry signals in the presence of artifact or interfering content and, in particular, to processes and associated structure for distinguishing useful physiological information from interfering content, that may be from a physiological or other source, and processing of such useful information to obtain parameter information, such as oxygen saturation, pulse rate and/or a plethysmographic waveform. The invention includes processes and associated structure for assessing the validity of received signals independent of the shape and waveform of a spectrum, for example, using fundamental and harmonic phase information, red and infrared spectral energy information (e.g., tracked over time) and combinations of the above.

## BACKGROUND OF THE INVENTION

Current pulse oximeters generally obtain two signals derived from the attenuation of red and infrared light signals as they are passed through a patient tissue site, typically a finger, nasal septum or ear lobe. A number of processing methods have been developed in the industry in both time and frequency domains to obtain both pulse rate information and the oxygen content (SpO2) level of the arterial blood from the attenuated red and infrared light signals. The attenuated red and infrared signals show a pulsing waveform that is related to the heart rate of the patient. These time domain signals, usually after some filtering, are used for display of the pulse cycle and are known as plethysmographic signals.

Measurement artifact including motion artifact and other noise as well as certain information of physiological origin, can interfere with accurate identification and measurement of a pleth and, therefore, with accurate determination of pulse rate and arterial oxygen saturation. A number of approaches have been developed to address such measurement artifact. Early approaches in this regard attempted to compensate for measurement artifact to a large extent without removing artifact from the signal under analysis. For example, such approaches included certain averaging techniques that damped measurement spikes associated with noise episodes. Later approaches involved weighted averaging whereby individual measurements were weighted in accordance with an objective confidence level related to the likely presence or absence of noise. These approaches reduced, to some degree, the effects of artifact, but also had limitations, especially in the cases of low perfusion and certain motion conditions.

More recent approaches have attempted to reject motion rather than compensate in the calculations. These approaches generally involve identifying some basis for distinguishing the pleth from measurement artifact and then controlling a filter in accordance with this basis. These approaches include saturation based filtering and cardiac based filtering.

Saturation based approaches generally assume that motion can be distinguished from instantaneous arterial absorption changes without knowing the patient's heart rate. For example, it may be assumed, in accordance with a saturation based approach that motion artifact is associated with the movement of venous blood and, as a result, certain correlation relationships between the red and infrared channel signals can be used to directly model and remove motion artifact.

Cardiac based approaches generally assume that artifact is random in spectral content or at least occurs to a significant extent in frequency bands separate from the patient's heart rate. Therefore, if heart rate can be accurately tracked, artifact can be addressed by filtering around the heart rate and, perhaps, one or more harmonics thereof. Additionally, some oximeter systems are designed to process signals differently during motion episodes than during periods of little or no motion. Such systems monitor certain parameters believed to distinguish motion episodes from periods of clean signals. That is, the signal is analyzed to estimate a magnitude of artifact present. In this manner, specialized motion processing can be limited to periods of potential motion. Periods of clean signals may be processed without concern that good information will be lost due to motion processing. In one system, implemented by Datex-Ohmeda, Inc., a further distinction is made between clinical and severe motion for optimized motion processing. Specifically, the pulsatile or AC portion of the signal is used for oxygen saturation calculations where motion is deemed low or clinical, but the slowly varying offset or DC portion of the signal is used, together with previously calculated values, for severe motion environments.

Another proposed motion solution makes multiple alternative calculations of the oxygen saturation and then arbitrates between the resulting values to select a value deemed best. Specifically, one calculation of arterial oxygen saturation is obtained in conventional fashion using substantially unfiltered data, and another is obtained by employing frequency based filtering for discriminating between the desired pleth and motion artifact. Certain assumptions are then used to select between the candidate values.

## SUMMARY OF THE INVENTION

It has been recognized that, due to the difficult nature of perceiving photoplethysmographic signals through artifact, it is useful to identify and characterize artifact environments and to employ processing techniques dependent on such artifact environments. In particular, in some such environments, it may be preferred to process a current AC portion of the signal to obtain the desired parameter information whereas, in other environments, it may be preferred to process a filtered AC portion of the signal or to process a DC portion of the signal, to use previously obtained signal portions or to otherwise employ information in place of or in addition to the current AC information. Also, different processing domains or algorithms may be employed depending on the artifact environment. In this regard, the signal may be analyzed to characterize the signal as likely including useful physiological information or likely including substantial interfering content, and to quantify or otherwise analyze such characteristics. Such an analysis may be conducted, for example, to validate a signal for further processing, to select a processing regime, to isolate a signal portion of interest from artifact, to generate a confidence factor, to modify the signal or filtering/processing parameters, to generate an oximeter output or other purposes.

In accordance with the present invention, information independent of a shape and waveform of a spectrum of a pulse oximeter signal is used to selectively process the signal. A number of processing techniques have heretofore been proposed or implemented for analyzing pulse oximeter signals with respect to distinguishing desired physiological signals from artifact. These have often involved analyzing the shape or waveform of a power spectrum or spectra. For example, the detector signal of the pulse oximeter is generally separated into red and infrared channels. These time-based channel signals are then typically transformed into the frequency domain and power spectra are generated. Parameters related to the shape of one or both of these spectra have been utilized to distinguish useful physiological components of interest from interfering information.

For example, the shape and/or spacing of spectral peaks may be used to determine whether a signal under analysis is likely a clean signal or an artifact affected signal. Different processing may be used depending on the result of this analysis. In other systems, the red and infrared spectra have been analyzed to determine parameters related to the correlation of the signals. Such analyses are, in effect, comparative analyses of the shapes or waveforms of the spectra. The result may be used, for example, to detect probe-off conditions, to selectively filter artifact from a desired pulsatile signal, or otherwise to identify and/or discriminate artifact.

However, distinguishing useful physiological information from interfering information based on spectral shape or waveform information can be problematic for a number of reasons. First, certain artifact can have significant power in frequency bands that overlap the physiological signal. As a result, associated spectral shape or waveform algorithms may fail to distinguish useful information from interfering information in a manner that results in overinclusion of interfering information or artifact affected signals. Moreover, certain physiological conditions, such as a rapidly changing pulse or arrhythmia, may mimic interfering effects as viewed in the spectral domain. Consequently, associated spectral shape or waveform algorithms may fail to distinguish useful physiological information from interfering information in a manner that results in underinclusion of useful physiological information. It will be appreciated that other difficulties exist in this regard.

Thus, in accordance with one aspect of the present invention, a method and apparatus ("utility") is provided for distinguishing useful physiological information from interfering information independent of the shape or waveform of a spectrum. The utility involves: obtaining a time-based signal representative of optical signals potentially including physiological information based on interaction with a patient; performing a transform on the time-based signal to obtain frequency-related information; performing an analysis on the frequency related information to obtain a value independent of a shape and waveform of a spectrum of the time-based signal; and using the value to determine a quality of the signal in relation to physiological information content and/or artifact content.

For example, the step of obtaining a time-based signal may involve operating a processor to receive digital information corresponding to the red and infrared channels of a detector of a pulse oximeter system. In this regard, the detector signal may be amplified, filtered, converted to digital form and otherwise conditioned upstream from the processor or certain of these functions may be performed in whole or in part in the logic of the digital processing unit. It will be appreciated that the time-based signal is generally a timed series of values corresponding to the detected intensity of the optical signals. The transform may be a Fast Fourier Transform or the like for converting a time-based signal into the frequency domain. Depending on the implementation, a power spectrum may be computed or the transform frequency domain information may be used without computing a power spectrum.

Various types of analyses may be performed in accordance with the present invention including various multi-component analyses. In one implementation, such multiple components include relative amplitude or power measures of the red and infrared spectra. For example, the ratio of the amplitude of the fundamental peak in the red spectrum and the amplitude of the fundamental peak in the infrared spectrum may be tracked over time. It has been observed that this ratio remains relatively constant in the case of useful physiological information, but tends to be more erratic in the case of interfering information. Thus, by tracking such a ratio or related values, a reliable indication can be obtained that the signal includes useful physiological information even though the shape or waveform of the spectrum may vary, for example, due to changing pulse rate or arrhythmia. Thus, the corresponding signal can be validated for use in particular processing regimes for calculating physiological parameters of interest.

Alternatively, the multi-component analysis may involve consideration of the phase of the fundamental and harmonic components. Specifically, the transform frequency information can be utilized without computing a power spectrum to retain phase information. The phase of the first harmonic component can then be measured in relation to the fundamental frequency to obtain relative phase information. Such phase information can be used to distinguish signals that likely include useful physiological information from signals that may be artifact affected. Additionally or alternatively, such relative phase information may be used to more accurately display a plethysmographic waveform generated using information that has been filtered by a bandpass filter to selectively pass the fundamental frequency of the detector signal. It will be appreciated that other uses are possible for such phase information.

It has further been recognized that measurement artifact including motion artifact is manifested in a variety of artifact types and that the assumptions underlying various motion processing techniques may be more or less valid depending on the particular type of artifact under consideration. For example, the assumptions underlying cardiac based motion rejection, including the assumption that motion artifact will occur to a significant degree outside of the frequency band of the patient's pulse, may not yield the desired level of motion rejection under certain motion conditions such as certain periodic or tapping motions by the patient. Similarly, the assumptions underlying saturation based motion, including the assumptions regarding certain correlation relationships between signal components, may not yield the desired level of motion rejection performance under certain circumstances. Rather, different types of artifact processing may be indicated for different types of measurement artifact.

Moreover, it has been recognized that, in certain cases, frequency domain harmonic phase information can be used to differentiate between signals indicative of patient artifact and those actually indicative of patient physiology. It has also been recognized that, in certain cases, spectral domain peak information can also be used to differentiate between signals indicative of patient artifact and those actually indicative of patient physiology. Therefore, in accordance with the present invention, AC component validity can be evaluated using such harmonic phase information and/or such relative spectral domain peak information.

In accordance with one aspect of the present invention, a utility provides physiological parameter information regarding a patient in the presence of measurement artifact based on verification of the presence or absence of the artifact, e.g., using validity analysis as described above. The physiological information may include, for example, pulse rate information, a pulsatility index, arterial blood oxygen saturation information, a plethysmographic waveform, respiration rate information, Mayer Wave related information and/or blood pressure/volume related information. Such information is preferably obtained noninvasively, e.g., via a pulse oximeter. The measurement artifact may be any of various noise or other undesired components that potentially interfere with such measurements including, for example, motion artifact relating to movement by or of the patient and irregular cardiac activity (e.g., arrhythmia).

In accordance with another aspect of the present invention, a utility is provided for differentiating between patient artifact and arrhythmia. As noted, in some circumstances known techniques are properly able to distinguish between patient artifact and patient physiology, but not in others. One particular circumstance in which difficulty arises is when a patient has arrhythmia. Often arrhythmia presents such that known pulse oximetry methods determine that the arrhythmia is artifact, when, in fact, it is patient physiology. It has been found that using phase validity measurements, spectral validity measurements, or a combination of the two can help distinguish between arrhythmia and artifact. Therefore, in one embodiment of the present invention a method is provided for differentiating between patient artifact and arrhythmia by placing an pulse oximeter in optical communication with the tissue of a patient, transmitting first and second optical signals of different wavelength through the tissue of a patient, detecting the transmitted first and second optical signals over a period of time, and evaluating whether the detected optical signals are indicative of artifact or arrhythmia using an arrhythmia determination model. In a preferred embodiment, the arrhythmia determination model is one of the above described spectral validity measurement, phase validity measurement, or a combinations thereof.

## BRIEF DESCRIPTION OF THE DRAWINGS

For a more complete understanding of the present invention and further advantages thereof, reference is now made to the following Detailed Description, taken in conjunction with the drawings, in which:

FIG. 1 is a block diagram of one embodiment of a pulse oximeter in which a cepstral domain plethysmographic signal processing method in accordance with the present invention may be implemented;

FIG. 2 is a block diagram showing one implementation of a method for processing plethysmographic signals via the cepstral domain in accordance with the present invention;

FIG. 3A is a plot showing typical red and infrared time domain plethysmographic input signals to be processed in accordance with the steps of FIG. 2;

FIG. 3B is a plot showing the Spectrum and Cepstrum for the red plethysmographic input signal of FIG. 3A after processing in accordance with the steps of FIG. 2;

FIG. 3C is a plot showing the Spectrum and Cepstrum for the infrared plethysmographic input signal of FIG. 3A after processing in accordance with the steps of FIG. 2;

FIG. 3D is a plot showing a typical infrared time domain plethysmographic input signal wherein the pulse oximeter probe is not transmitting properly through a patient tissue site (e.g., where the probe is removed from the patient's finger);

FIG. 3E is a plot showing the Spectrum and Cepstrum for the infrared plethysmographic signal of FIG. 3D after processing in accordance with the steps of FIG. 2;

FIG.4 is a block diagram showing another implementation of a method for processing plethysmographic signals via the cepstral domain in accordance with the present invention;

FIG. 5A is a plot showing typical red and infrared time domain plethysmographic input signals to be processed in accordance with the steps of FIG. 4;

FIG. 5B is a plot showing differentiated waveforms obtained from the typical red and infrared time domain plethysmographic input signals shown in FIG. 5A;

FIG. 5C is a plot showing red and infrared energy spectra corresponding to the typical red and infrared time domain plethysmographic input signals shown in FIG. 5A;

FIG. 5D is a plot showing red and infrared log spectra corresponding to the typical red and infrared time domain plethysmographic input signals shown in FIG. 5A;

FIG. 5E is a plot showing red and infrared cepstrums corresponding to the typical red and infrared time domain plethysmographic input signals shown in FIG. 5A;

FIG. 5F is a plot showing frequency domain filtered red and infrared plethysmographic waveforms corresponding to the typical red and infrared time domain plethysmographic input signals shown in FIG. 5A;

FIG. 6A is a plot showing typical red and infrared time domain plethysmographic input signals to be processed in accordance with the steps of FIG. 4 that include motion induced noise components at a main motion frequency of about 200 bpm;

FIG. 6B is a plot showing differentiated waveforms obtained from the typical red and infrared time domain plethysmographic input signals shown in FIG. 6A;

FIG. 6C is a plot showing red and infrared energy spectra corresponding to the typical red and infrared time domain plethysmographic input signals shown in FIG. 6A;

FIG. 6D is a plot showing red and infrared log spectra corresponding to the typical red and infrared time domain plethysmographic input signals shown in FIG. 6A;

FIG. 6E is a plot showing red and infrared cepstrums corresponding to the typical red and infrared time domain plethysmographic input signals shown in FIG. 6A;

FIG. 6F is a plot showing frequency domain filtered red and infrared plethysmographic waveforms corresponding to the typical red and infrared time domain plethysmographic input signals shown in FIG. 6A;

FIG. 7 is a block diagram showing another implementation of a method for processing plethysmographic signals via the cepstral domain in accordance with the present invention;

FIG. 8 is a plot showing typical red and infrared time domain plethysmographic input signals to be processed in accordance with the steps of FIG. 7;

FIGS. 9A-9B are plots of an energy spectrum and a cepstrum corresponding to an exemplary plethysmographic signal;

FIGS. 10A-10B are plots of exemplary time domain, spectral domain, cepstral domain, and DC tracking based SPO2 estimates over a period of time;

FIGS. 11A-11B are plots of showing successive frames of exemplary spectrums and cepstrums corresponding to a plethysmographic signal in which various levels of motion are present over the time periods covered by the successive frames;

FIG. 12 is a flowchart illustrating a process for tailoring signal processing based on an artifact classification in accordance with the present invention;

FIG. 13 is a block diagram showing a system for implementing the process of Fig. 12;

FIG. 14 is a schematic diagram showing further details of the system of Fig. 13;

FIGS. 15A -15B illustrate red and infrared spectra of useful physiological signals and interfering signals, respectively, as observed in accordance with the present invention;

FIGS. 16A - 16B illustrate observed R-ratio patterns that can be distinguished in accordance with the present invention; and

FIGS. 17 - 18 are flowcharts illustrating processes for distinguishing between useful physiological information and interfering information in accordance with the present invention.

**DETAILED DESCRIPTION**

In the following description, a pulse oximetry system is first described, followed by certain motion processing. Thereafter, specific functionality for distinguishing useful physiological information from interfering information based on analyses substantially independent of spectral shape and waveform is described.

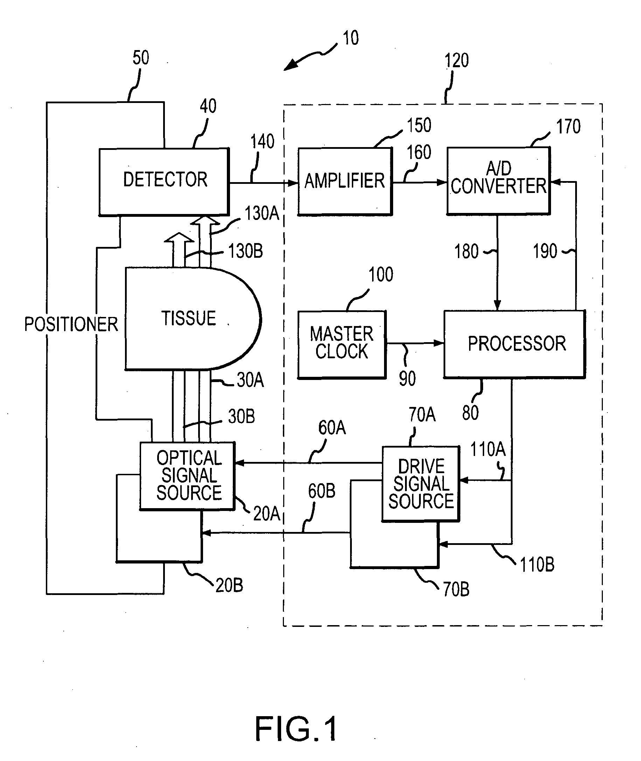

Referring now to Fig. 1, there is shown a block diagram of one embodiment of a pulse oximeter 10 in which a multi-domain plethysmographic signal processing method in accordance with the present invention may be implemented. The pulse oximeter 10 is configured for use in determining the pulse rate of a patient as well as one or more blood analyte levels in the patient, such as an SPO2 level. It should be appreciated that a multi-domain plethysmographic signal processing method in accordance with the present invention may be implemented in pulse oximeters that are configured differently from the pulse oximeter depicted in FIG. 1 as well as in other environments wherein plethysmographic signals are processed in order to obtain desired information relating to patient physiological conditions from the plethysmographic signals.

The pulse oximeter 10 includes a pair of optical signal sources 20a, 20b for emitting a corresponding pair of light signals 30a, 30b centered at different predetermined center wavelengths $\lambda_1$, $\lambda_2$ through (or reflected from) a suitable tissue site of a patient and on to a detector 40 (e.g., a photo-sensitive diode). The optical signal sources 20a, 20b and detector 40 may be included in a positioning device 50, or probe, to facilitate alignment of the light signals 30a, 30b with the detector 40. For example, the positioning device 50 may be of clip-type or flexible strip configuration adapted for selective attachment to a suitable patient tissue site (e.g., a finger, an ear lobe, a foot, or the nose of the patient). The center wavelengths $\lambda_1$, $\lambda_2$ required depend upon the blood analyte level to be determined. For example, in order to determine an SPO2 level, $\lambda_1$ may be in the red wavelength range and $\lambda_2$ may be in the infrared wavelength range. It should be appreciated that the pulse oximeter 10 may be readily implemented with more optical signal sources (e.g., four) depending, for example, upon the number of different blood analyte levels to be measured.

The optical signal sources 20a, 20b are activated by a corresponding plurality of drive signals 60a, 60b to emit the light signals 30a, 30b. The drive signals 60a, 60b are supplied to the optical signal sources 20a, 20b by a corresponding plurality of drive signal sources 70a, 70b. The drive signal sources 70a, 70b may be connected with a digital processor 80, which is driven with a clock signal 90 from a master clock 100. The digital processor 80 may be programmed to define modulation waveforms, or drive patterns, for each of the optical signal sources 20a, 20b. More particularly, the digital processor 80 may provide separate digital trigger signals 110a, 110b to the drive signal sources 70a-d, which in turn generate the drive signals 60a, 60b. In this regard, the digital trigger signals 110a, 110b may be configured to provide for multiplexing of the drive signals 60a, 60b, and in turn the light signals 30a, 30b, in accordance with a multiplexing scheme (e.g., time division, frequency division, and/or code division multiplexing).

The drive signal sources 70a, 70b, processor 80 and clock 100 may all be housed in a monitor unit 120. While the illustrated embodiment shows the optical signal sources 20a, 20b physically interconnected with the positioning device 50 (e.g., mounted within the positioning device 50 or mounted within a connector end of a cable that is selectively connectable with the positioning device 50), it should be appreciated that the optical signal sources 20a, 20b may also be disposed within the monitor unit 120. In the latter case, the light signals 30a, 30b emitted from the optical signal sources 20a, 20b may be directed from the monitor unit 120 via one or more optical fibers to the positioning device 50 for transmission through the tissue site. Furthermore, the drive signal sources 70a, 70b may comprise a single drive signal generator unit that supplies each of the drive signals 60a, 60b to the optical signal sources 20a, 20b.

Transmitted light signals 130a, 130b (i.e., the portions of light signals 30a, 30b exiting the tissue) are detected by the detector 40. The detector 40 detects the intensities of the transmitted signals 130a, 130b and outputs a current signal 140 wherein the current level is indicative of the intensities of the transmitted signals 130a, 130b. As may be appreciated, the current signal 140 output by the detector 40 comprises a multiplexed signal in the sense that it is a composite signal including information about the intensity of each of the transmitted signals 130a, 130b. Depending upon the nature of the drive signals 60a, 60b, the current signal 140 may, for example, be time division multiplexed, wavelength division multiplexed, and/or code division multiplexed.

The current signal 140 is directed to an amplifier 150, which may be housed in the monitor unit 120 as is shown. As an alternative, the amplifier 150 may instead be included in a probe/cable unit that is selectively connectable with the monitor unit 120. The amplifier 150 converts the current signal 140 to a voltage signal 160 wherein a voltage level is indicative of the intensities of the transmitted signals 130a, 130b. The amplifier 150 may also be configured to filter the current signal 140 from the detector 40 to reduce noise and aliasing. By way of example, the amplifier 150 may include a bandpass filter to attenuate signal components outside of a predetermined frequency range encompassing modulation frequencies of the drive signals 60a, 60b.

Since the current signal 140 output by the detector 40 is a multiplexed signal, the voltage signal 160 is also a multiplexed signal, and thus, the voltage signal 160 is demultiplexed in order to obtain signal portions corresponding with the intensities of the transmitted light signals 130a, 130b. In this regard, the digital processor 80 may be provided with demodulation software for demultiplexing the voltage signal 160. In order for the digital processor 80 to demodulate the voltage signal 160, it is converted from analog to digital. Conversion of the analog voltage signal 160 is accomplished with an analog-to-digital (A/D) converter 170, which may also be included in the monitor unit 120. The A/D converter 170 receives the analog voltage signal 160 from the amplifier 150, samples the voltage signal 160, and converts the samples into a series of digital words 180 (e.g., eight, sixteen or thirty-two bit words), wherein each digital word is representative of the level of the voltage signal 160 (and hence the intensities of the transmitted light signals 130a, 130b) at a particular sample instance. In this regard, the A/D converter 170 should provide for sampling of the voltage signal 160 at a rate sufficient to provide for accurate tracking of the shape of the various signal portions comprising the analog voltage signal 160 being converted. For example, the A/D converter 170 may provide for a sampling frequency at least twice the frequency of the highest frequency drive signal 60a, 60b, and typically at an even greater sampling rate in order to more accurately represent the analog voltage signal.

The series of digital words 180 is provided by the A/D converter 170 to the processor 80 to be demultiplexed. More particularly, the processor 80 may periodically send an interrupt signal 190 (e.g., once per every eight, sixteen or thirty-two clock cycles) to the A/D converter 170 that causes the A/D converter 170 to transmit one digital word 180 to the processor 80. The demodulation software may then demultiplex the series of digital words 180 in accordance with an appropriate method (e.g., time, frequency and/or code) to obtain digital signal portions indicative of the intensities of each of the transmitted light signals 130a, 130b. In this regard, the demultiplexed digital signal portions comprise time domain plethysmographic signals corresponding to the center wavelengths $\lambda_1$, $\lambda_2$ (e.g., red and infrared) of the optical signal sources 20a, 20b. The red and infrared time domain plethysmographic signals may then be processed by the processor 80 to obtain desired patient physiological condition related information therefrom such as the patient's pulse rate and SPO2 level.

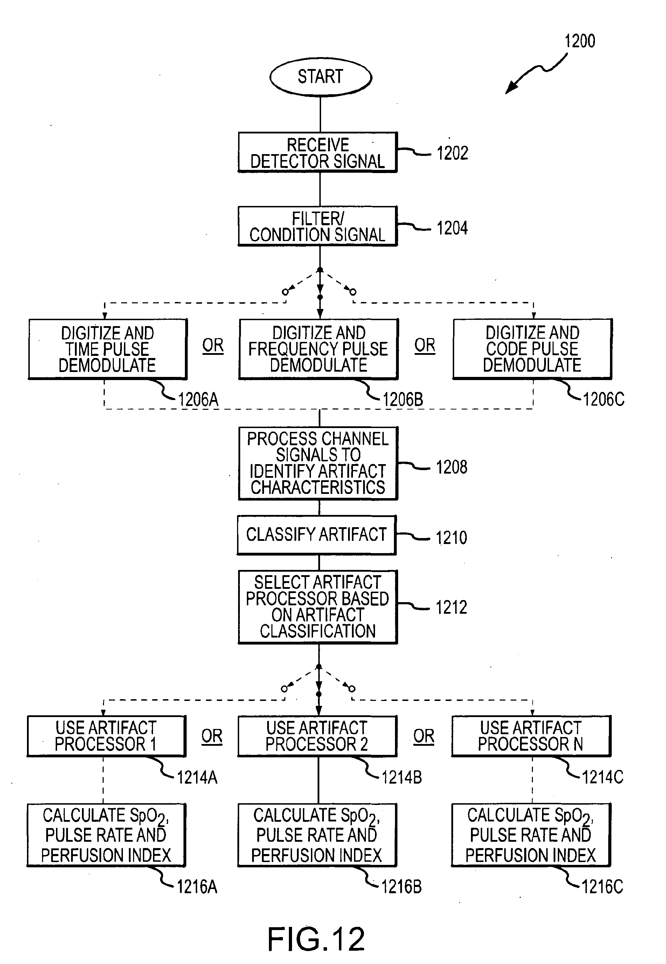

As noted above, significant advantages can be achieved by optimizing signal processing based on substantially current artifact conditions including patient motion conditions. Fig. 12 illustrates a process 1200 for analyzing and classifying potential artifact such that appropriate processing may be implemented. The process 1200 is initiated by receiving (1202) the detector signal, typically a multiplexed signal including at least red and infrared channels where oxygen saturation calculations are desired. In the illustrated process, the detector signal is then filtered, e.g., band-pass filtered (1204), to remove undesired components outside of frequency range of interest for the desired physiological parameter, amplified, converted to digital form and otherwise conditioned for subsequent processing.

The conditioned signal is then demultiplexed (1206A-C) to obtain differentiated channel information. In this regard, the detector signal may be time division multiplexed, frequency division multiplexed and/or code division multiplexed and an appropriate demultiplexing signal may be utilized to deconvolve the channel components. It will be appreciated that hybrid multiplexing arrangements may be utilized in this regard. For example, the sources may be pulsed in a nonperiodic fashion to define coded pulses and those codes may be selected such that the sources are not pulsed at the same time so as to provide time division multiplexing.

One or more of the channel signals may then be processed (1208) to identify artifact characteristics. Artifact can be identified and analyzed in a variety of different ways. For example, in the time domain, artifact may be reflected by distortion or obfuscation of the plethysmographic waveform ("pleth"). Excessive roughness of the pleth or spurious peaks may be observed in this regard. In the spectral and cepstral domains, artifact may be reflected, for example, as broadened peaks and/or peaks not associated with the fundamental frequency of the pleth and harmonics thereof. Examples of parameters that may be measured or tracked in this regard include variance of time domain data from a regression line over a sampling time window, the shapes of the pleths and variation thereof, variation of the ratio of the slopes of the red and infrared pleths in the time domain, correlation of the red and infrared signals, width of spectral or cepstral peaks, movement of the spectral or cepstral peaks, the presence, number and/or magnitude of spectral or cepstral peaks not associated with the fundamental frequency of the pleth or harmonics thereof, the relative powers of such peaks or of such peaks in relation to the fundamental peak and/or harmonics, information obtained from DC component analysis or other instrumentation, and relationships among such parameters. Such parameters can be measured to provide a fingerprint for the artifact environment.

Based on this information, the artifact can be classified (1210). This is preferably a clinically based decision. That is, the artifact characteristics can be compared to known artifact conditions observed in a clinical environment to associate the artifact characteristics with a particular known artifact condition, e.g., on a closest match basis such as by scoring. In this regard, the known artifact conditions may include tapping motion, irregular cardiac activity, and various types of characteristic infant motion. Many of these artifact conditions may have a cognizable fingerprint as reflected in some artifact parameter set. Certain processes for distinguishing artifact affected signals from "clean" signals or for validating a signal are discussed in more detail below. This matching process may be based on fixed algorithms established based on clinical data or may be guided by a heuristic engine employing fuzzy logic for optimized pattern recognition.

This classification is then utilized to select (1212) an appropriate artifact processor in the illustrated process 1200. Each of the processors may be, for example, a software module for executing a particular processing algorithm. Examples of different processors that may be utilized include processors for: determining a pulse rate based on an analysis of the time domain signal and using the pulse rate to tune an adaptive filter such as a single or multiple bandpass or notch filter; determining a pulse rate based on an analysis of the spectral or cepstral domain signal and using the pulse rate to tune an adaptive filter; applying an artifact compensation factor to a physiological component value determined from the detector signal substantially without artifact rejection within the relevant frequency range; selecting a set of DC component information for use in calculating a physiological parameter value; selecting a recent value of the desired physiological parameter and scaling the parameter based on DC tracking information; and selecting a sampling window position and size or an averaging filter time constant.

Thus, using a processor (1214A-C) a processor may entail accessing a selected data set for further processing, executing a selected algorithm for artifact rejection or compensation, employing a selected filter type, and/or employing selected filter coefficients. All of this may be based on theoretical and/or empirical considerations and, preferably, results in selection and use of a processor that provides desired results in relation to clinical data under similar artifact conditions. As shown, the selected artifact processor can be used in conjunction with an appropriate physiological parameter processor to calculate (1216A-C) a value for a physiological parameter such as SpO2, pulse rate or perfusion index.

The artifact analysis is preferably performed on a "raw" detector signal, i.e., prior to frequency based filtering within the frequency range of interest or other processing that may reject artifact components. In many cases, the physiological parameter calculation is performed on processed data resulting from processing by one of the artifact processors (1214A-C). In other cases, the raw pleth signals may be used, for example, with a motion compensation factor associated with an artifact processor (1214A-C). Thus, the artifact processors (1214A-C) may operate before, after or in parallel with the physiological parameter calculation modules. Additionally, as shown, only the selected artifact processor (1214A-C) is utilized to provide the desired output. However, as a matter of expediency, all or at least multiple ones of the processors (1214A-C) may process particular pleth data in parallel, e.g., concurrent with identification (1208) and classification (1210) of the artifact. For example, such parallel processing may be desirable to reduce display latency even though only one of the processors will provide the information used for a particular displayed result. This is readily contrasted to post calculation analysis, such as comparing candidate values of the physiological parameter, which require analysis of completed physiological parameter calculations to select a result deemed to be less affected by artifact.

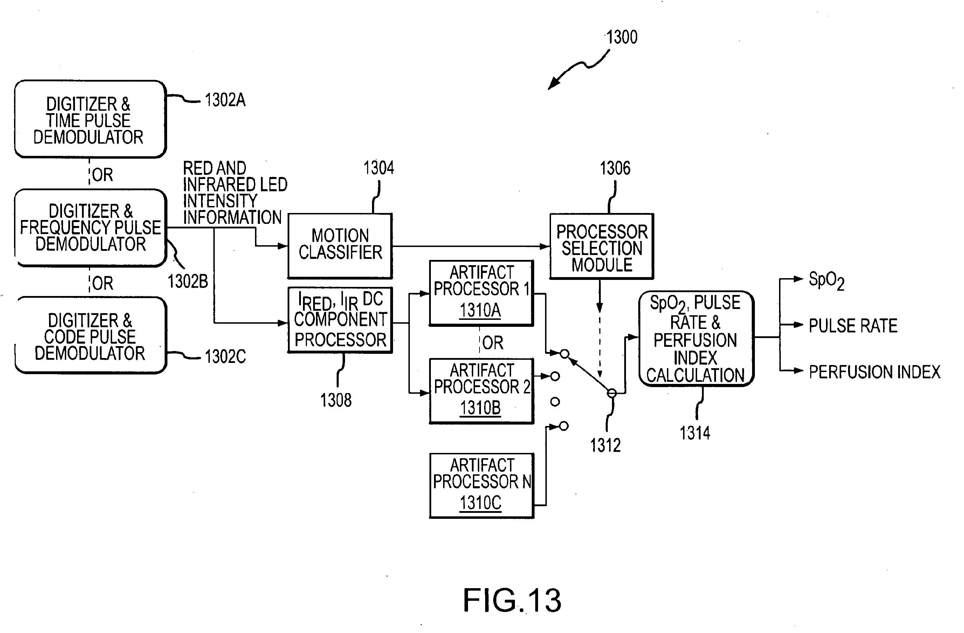

Fig. 13. is a component diagram of a signal processor 1300 for implementing the process of Fig. 12. The processor 1300 includes a digitizer and demodulator 1302A, 1302B or 1302C depending on the signal modulation scheme employed. In this regard, the illustrated processor may include unit 1302A in the case of time division multiplex implementations, unit 1302B in the case of frequency division multiplex implementations or unit 1302C in the case of code division multiplex implementations. The resulting digitized channel information, generally including both and red and infrared channel information, is provided both to motion classifier 1304 and processor 1308. The motion classifier 1304 analyzes the digital data as described above to identify artifact characteristics and classify the artifact, in this case, classifying motion artifact. The processor 1308 processes the intensity signal for each of the channels to provide the inputs required by the artifact processors 1310A-C. For example, certain artifact processors may require determination of a pulse rate or artifact frequency value for tuning a filter. Processor 1308 may perform a variety of calculations in this regard. The processors 1310A-C then process the channel signals for artifact rejection or compensation as discussed above. As illustrated graphically by switch 1312, a selected one of the artifact processors 1310A, 1310B or 1310C is used in conjunction a physiological parameter calculation module 1314 to provide the desired output.

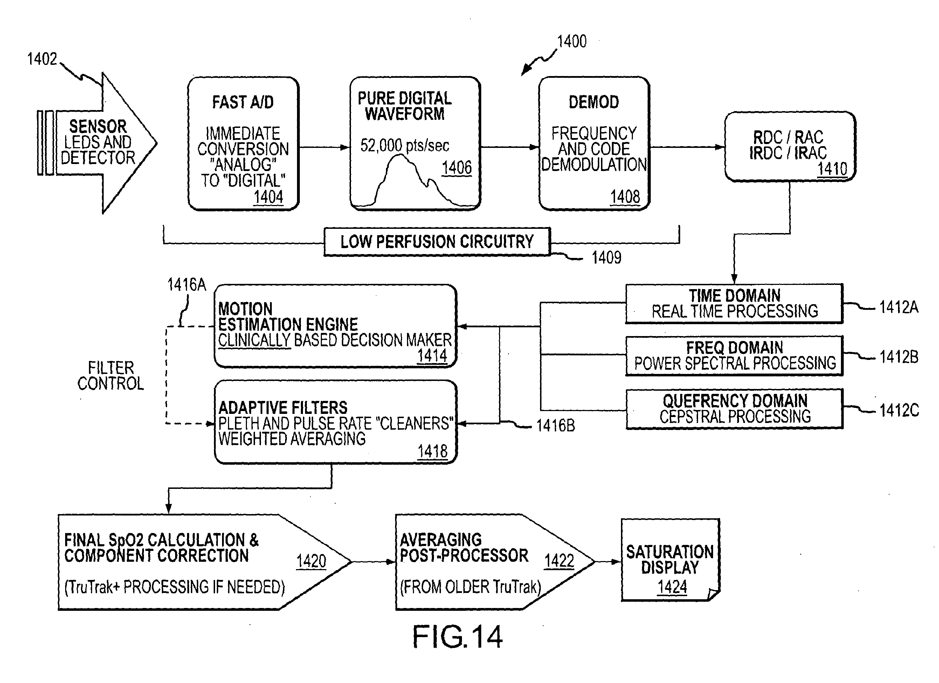

Further details of this implementation are illustrated in the schematic diagram of Fig. 14. As shown, the sensor 1402, which includes the LED sources and a detector, provides an input to a fast analog-to-digital converter 1404. The converter 1404 outputs a digital waveform, for example, at a sampling rate of approximately 46,000 to 52,000 points per second. This digital waveform is provided to a demodulator 1408 that may execute time, frequency and/or code division demultiplexing. The converter 1404 and demodulator 1408 define low perfusion circuitry 1409. It will be appreciated that the fast analog-to-digital converter in conjunction with certain demodulation schemes for improved noise rejection and signal identification provide enhanced results for patients with low perfusion.

The resulting channel signals may be separated into AC and DC components by channel processing module 1410 and various values may be calculated from this raw signal for use in further analysis. The channel signals may then be provided to processors 1412A-C for time domain, spectral domain and cepstral domain processing. Such processing may be utilized both for artifact classification and for motion rejection or compensation. The output from the processors 1412A-C is provided to a motion estimation engine 1414 and an adaptive filter 1418 in the illustrated implementation. The motion estimation engine measures and classifies motion artifact and provides a filter control output 1416A, that operates in conjunction with filter control information 1416B from processors 1412A-C, to tune the adaptive filters 1418, for example, via appropriate filter coefficient selection. In this regard, the adaptive filters may include a multiple bandpass filter that can be tuned to the patient's pulse rate. Such filtering can provide a pleth waveform with substantial motion rejection which can be used for improved pulse rate, SpO2 perfusion index and other calculations. In addition to setting filter coefficients, the filter control outputs 1416A and B may be used to select the placement and size of sample windows as well as provide weighting values based on motion levels.

The output from the adaptive filters 1418 can then be used by physiological component module 1420 to calculate SpO2 or other parameter values and provide an optimized pleth waveform. Additionally or alternatively, module 1420 may calculate values based on the DC components of the pleth or based on a recently calculated physiological parameter value as corrected based on DC tracking. Such an additional or alternative DC tracking calculation is disclosed, for example, in U.S. Pat. No. 6,839,582 entitled "Pulse Oximetry Method and System with Improved Motion Correction," which is incorporated herein by reference. In cases of extreme motion, the module 1420 may determine that physiological parameter values cannot be reliably calculated and may cause the display to be dashed. The output from module 1420 is provided to an averaging post processor 1422. The averaging post processor averages physiological parameter values over a time window to prevent excessive display flicker. The resulting average value is then provided to the saturation display 1424.

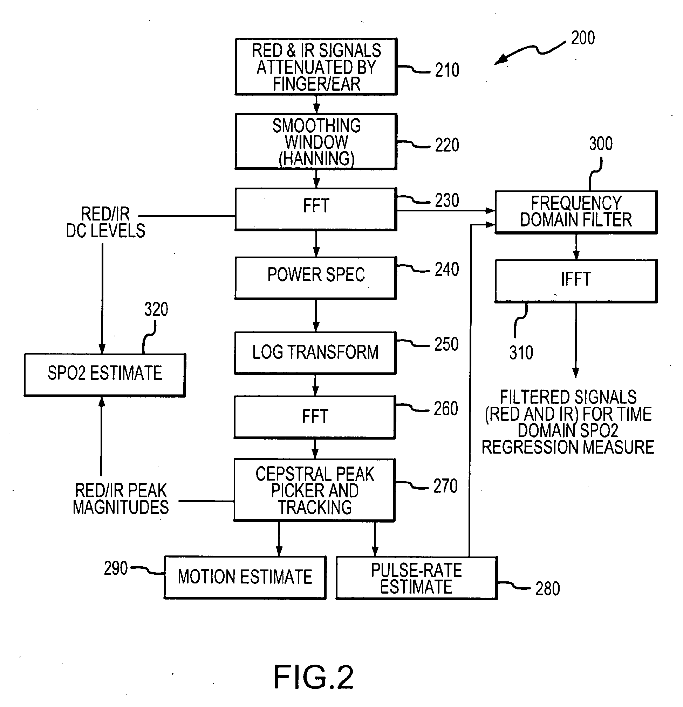

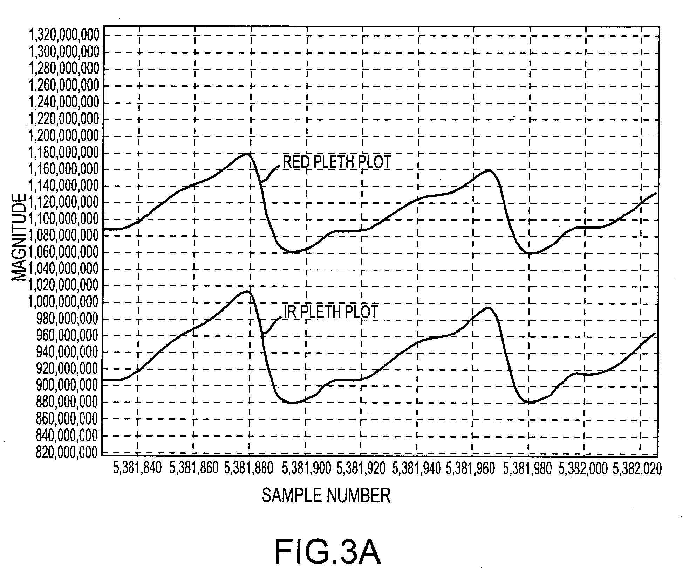

A multi-domain analysis, for example, including a cepstral domain analysis may be used for optimized artifact processing. Referring now to FIG. 2 there is shown a block diagram illustrating one implementation of a method (200) for processing the red and infrared time domain plethysmographic signals via the cepstral domain to obtain desired information relating to patient physiological conditions such as patient pulse rate and blood analyte level (e.g., SPO2) information. The cepstral domain plethysmographic signal processing method (200) begins with obtaining (210) two digitized time domain plethysmographic signals such as red and infrared plethysmographic signals. In this regard, typical red and infrared time domain plethysmographic signals that have been sampled at 50 Hz are shown in FIG. 3A. The cepstral domain processing method (200) is particularly suited for implementation in software executable by the digital processor 80 of a pulse oximeter 10 such as described above in connection with FIG. 1. In other embodiments, the cepstral domain processing method (200) may be configured for processing non-digitized plethysmographic signals and may be implemented in appropriate hardware components. Furthermore, the cepstral domain processing method (200) may be configured for simultaneously processing more than two plethysmographic signals.

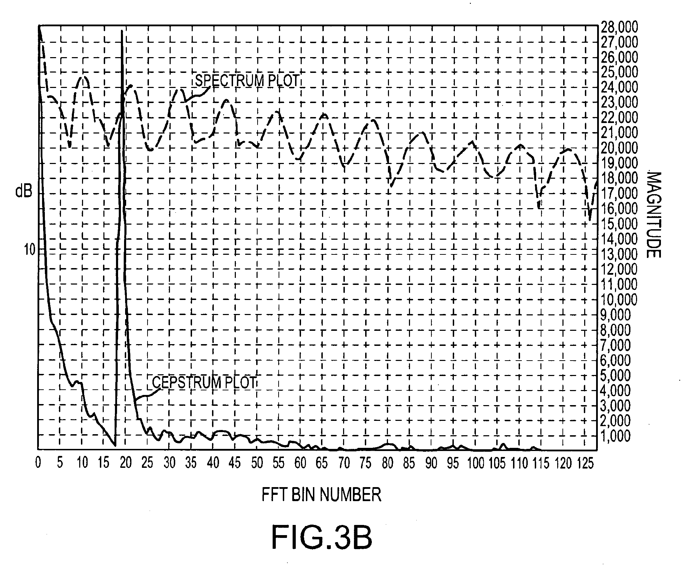

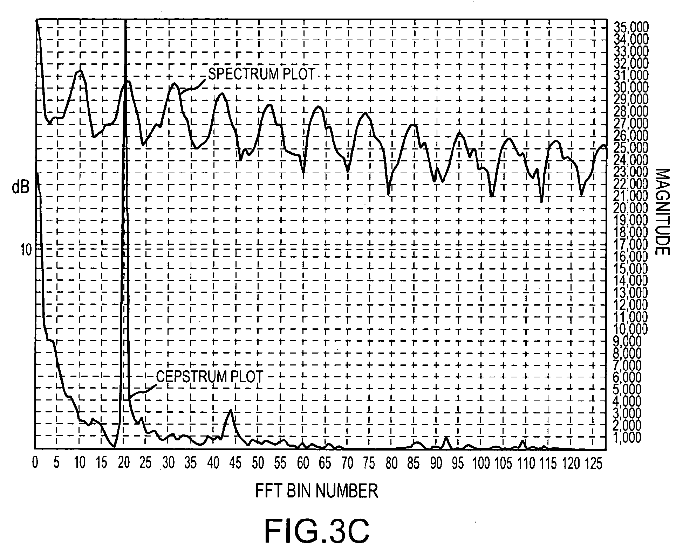

A suitable smoothing window function (e.g., Hanning, Hamming, Kaiser) is applied (220) to the digitized time domain plethysmographic signals to smooth the signals. Smoothing the digitized time domain plethysmographic signals achieves improved frequency estimation. After the signals are smoothed, a first Fourier transformation operation is performed (230) on the signals to transform the red and infrared plethysmographic signals from the time domain to the frequency domain. Since there are two primary signals (the red and infrared inputs), it is convenient to perform the first Fourier transformation of the signals in parallel using a complex Fast Fourier Transform (FFT) procedure. If desired, the results of the FFT calculations may be appropriately scaled (e.g., by dividing by the number of points used in the FFT calculations) to help prevent floating point overflow errors in subsequent computations. After the first stage FFT is performed, respective power spectrums are computed (240) from the frequency domain red and infrared plethysmographic signals. In this regard, the power spectrums may be computed (240) by squaring and summing the appropriate real and imaginary frequency components of the red and infrared frequency domain plethysmographic signals. Power spectrums of the typical red and infrared plethysmographic signals after the first stage FFT are shown in FIGS. 3B and 3C, respectively.

After the power spectrums are computed, a log-like or companding function is applied (250) to the red and infrared power spectrums. Application of the log-like or companding function suppresses smaller noise components and emphasizes the prominent harmonics so that periodicity in the spectrum is more easily extracted. A second Fourier transformation operation is then performed (260) on the log transformed power spectrums to transform the signals to the cepstral domain. In this regard, it is convenient to perform the second-stage Fourier transformation of the log scaled power spectrums in parallel using a complex Fast Fourier Transform (FFT) procedure. If desired, the results of the second-stage FFT calculations may be appropriately scaled in a manner similar to scaling done on the results of the first-stage FFT calculations. The cepstrums of the typical red and infrared plethysmographic signals obtained after the second stage FFT are also shown in FIGS. 3B and 3C, respectively.

Once the red and infrared cepstrums are obtained, the separate red and infrared cepstrums are then examined (270) for peaks associated with the pulse rate of the patient. In this regard, the most prominent (i.e., largest amplitude) peak in each cepstrum may be identified. The location of the most prominent peak in each cepstrum provides an indication of the fundamental frequency of the plethysmographic waveform from which the cepstrum is obtained. Since the fundamental frequency of a plethysmographic waveform is proportional to the patient's pulse rate, the pulse rate of the patient may be estimated (280) from one or both of the cepstrums. For example, the most prominent peak in the red cepstrum of FIG. 3B occurs at around the 20th bin of the FFT spectrum corresponding to a cepstral based pulse rate estimate of approximately 65 beats-per-minute. It should be noted that this estimate differs slightly from a conventional time domain based estimate obtained from the time domain red plethysmographic waveform shown in FIG. 3A of 61 beats-per-minute. Pulse-rate estimates may be obtained from one or both the red and infrared cepstrums and one of the separate estimates may be selected, e.g., based on artifact related rules, in order to obtain a single estimate of the patient's pulse rate. Further, while it is possible to estimate the patient's pulse rate based only on information from one or both of the cepstrums, a time domain based estimate of the patient's pulse rate may be used in certain artifact environments or for initial identification purposes and to support subsequent tracking of the cepstral peak (Quefrency) associated with the pulse rate.

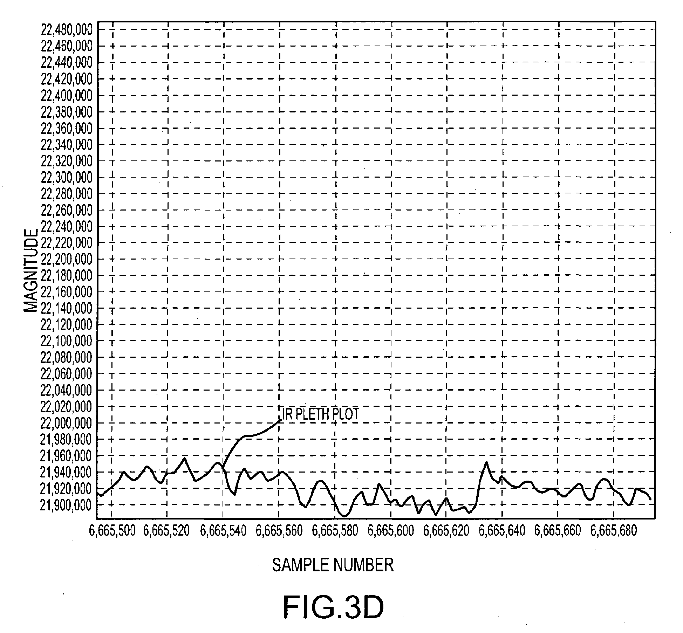

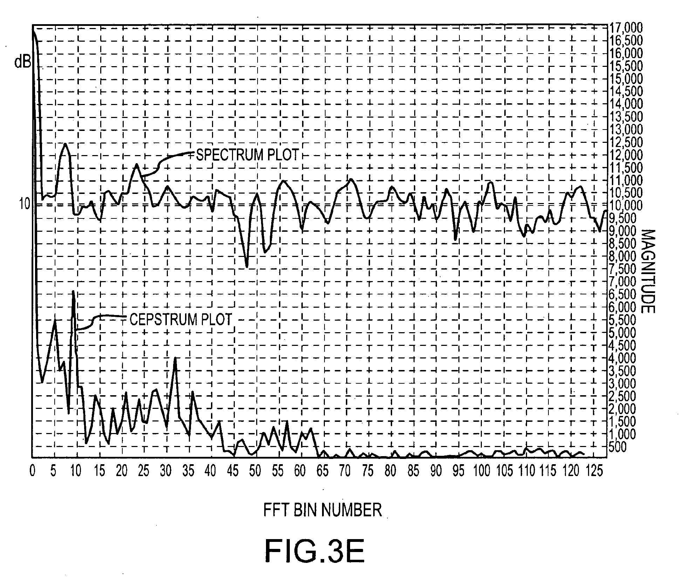

In some cases, there may not be a prominent peak in one or both of the cepstrums. For example, FIG. 3D shows an infrared time domain plethysmographic signal typical of the situation where there is no physiological signal condition (e.g., where the plethysmographic probe has been removed from the patient's finger), and FIG. 3E shows the infrared power spectrum and cepstrum obtained for the infrared time domain plethysmographic signal of FIG. 3D. While the power spectrum of FIG. 3E differs somewhat from a power spectrum that is typical of a patient physiological signal condition such as the power spectrums shown in FIGS. 3B and 3C, the lack of a patient physiological signal condition is particularly apparent from examination of the cepstrum since there is no prominent peak present in the cepstrum of FIG. 3E as compared with the quite prominent cepstral peaks in FIGS. 3B and 3C. Such an analysis may be used to identify probe-off conditions and provide appropriate alarms.

In addition to examining the cepstrums for peaks associated with patient pulse rate, in step (270) the red and infrared cepstrums may be examined for peaks associated with motion artifact. Typically, peaks in the red and infrared cepstrums that are associated motion artifact will be less prominent than the peaks associated with the patient pulse rate. The location(s) of less prominent peaks in each cepstrum provide an indication regarding motion artifact present in the plethysmographic waveform from which the cepstrum is obtained, and based on this information the frequencies and classification of motion artifact present in the red and infrared plethysmographic signals may be estimated (290).

Once an estimate of the pulse rate is obtained, the pulse rate information may be used to tune a filter to remove noise and motion artifact from the input red and infrared signals. This may be done via an adaptive bandpass filter applied in the time domain to the red and infrared signals where the cut off frequencies are determined by the pulse frequency which is identified, for example, in the spectral or cepstral domain. Alternatively, as is shown in the embodiment of FIG. 2, the frequency domain red and infrared plethysmographic signals may be filtered (300) in the frequency domain after the first stage FFT with a frequency domain filter constructed using the pulse frequency information obtained from the cepstral domain. An inverse fast Fourier transform (IFFT) operation may be performed (310) on the filtered frequency domain signals to obtain filtered time domain red and infrared plethysmographic signals for use in subsequent measures such as a regression based SPO2 estimation which may use the time domain version of the red and infrared inputs signals. Noise removal from the red and infrared signals improves subsequent measures such as regression based SPO2 estimation.

Additionally, the information in the spectral and cepstral domains may be selectively used to derive an SPO2 measure. The overall DC levels of the red and infrared plethysmographic signals can be determined from the first stage spectrums and the relative magnitudes of the cepstral peaks corresponding to the pulse rate frequency may be used to obtain a measure of the AC levels of the red and infrared plethysmographic signals. In this regard, the following computation may be utilized:

$$R' = AC(cepstral\text{-}red) \, / \, DC(spectral\text{-}red) \, / \, AC(cepstral\text{-}IR) \, / \, DC(spectral\text{-}IR)$$

or, expressed in an alternative manner:

$$R' = AC(cepstral\text{-}red) \, / \, DC(spectral\text{-}red) \, * \, DC(spectral\text{-}IR) \, / \, AC(cepstral\text{-}IR)$$

where $AC(cepstral\text{-}red)$ is the AC level of the red plethysmographic signal obtained from the red cepstrum, $DC(spectral\text{-}red)$ is the DC level of the red plethysmographic signal obtained from the red spectrum, $AC(cepstral\text{-}IR)$ is the AC level of the infrared plethysmographic signal obtained from the infrared cepstrum, and $DC(spectral\text{-}IR)$ is the DC level of the infrared plethysmographic signal obtained from the infrared spectrum. The derived measure R' may then be used to estimate (320) the patient's SPO2 level in a manner similar to known regression techniques where AC and DC estimates are obtained from the time domain red and infrared signals. An example of such a known regression technique is described in United States Patent No. 5,934,277 entitled "SYSTEM FOR PULSE OXIMETRY SPO2 DETERMINATION", the entire disclosure of which is incorporated herein.

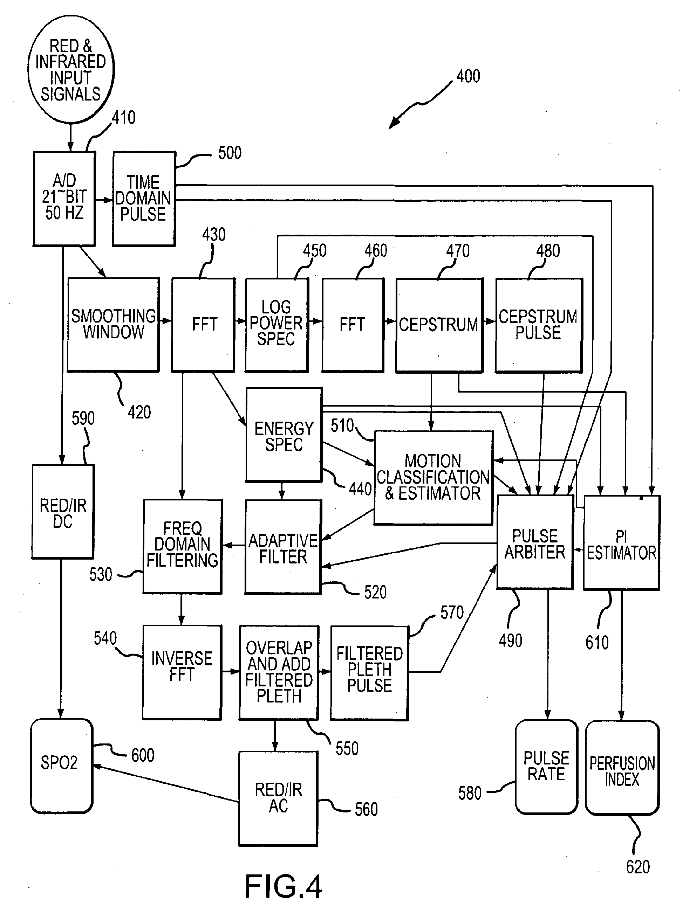

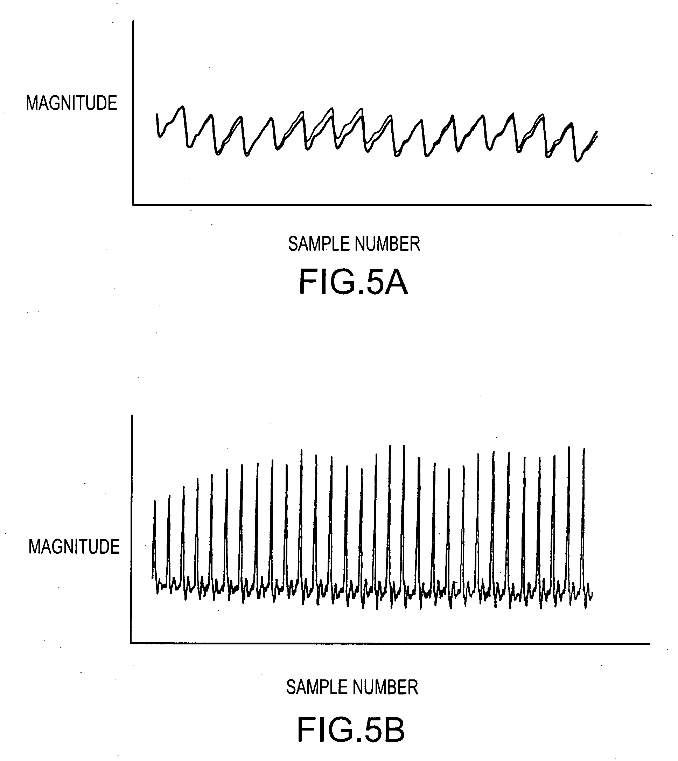

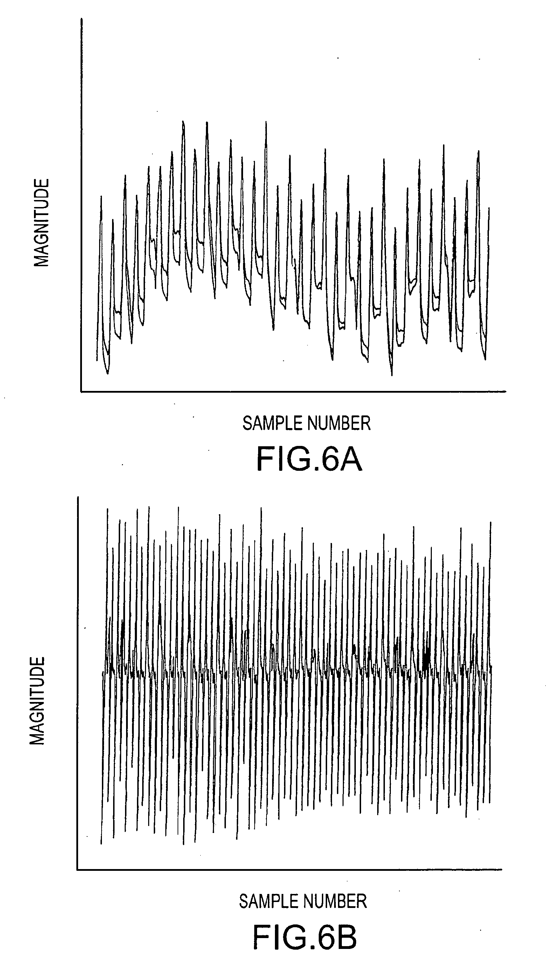

Referring now to FIG. 4 there is shown a block diagram illustrating another implementation of a method (400) for processing the red and infrared time domain plethysmographic signals via multiple domains to obtain desired information relating to patient physiological conditions such as patient pulse rate and blood analyte level (e.g., SPO2) information. In this case, inputs from a selected one of multiple domains may be used to tune a bandpass filter based on the artifact environment. The cepstral domain plethysmographic signal processing method (400) shown in FIG. 4 proceeds in a manner similar to the method (200) shown in FIG. 2. In this regard, two continuous time domain plethysmographic signals such as red and infrared plethysmographic signals are digitized (410) by sampling the signals at a suitable frequency. Typical red and infrared time domain plethysmographic signals that have been sampled at 50 Hz are shown in FIGS. 5A and 6A, with the signals of FIG. 6A including motion artifact. As with the method (200) of FIG. 2, the multi-domain processing method (400) is particularly suited for implementation in software executable by the digital processor 80 of a pulse oximeter 10 such as described above in connection with FIG. 1, and in other embodiments, the multi-domain processing method (400) may be configured for processing non-digitized plethysmographic signals and may be implemented in appropriate hardware components. Furthermore, the multi-domain processing method (400) may be configured for simultaneously processing more than two plethysmographic signals.

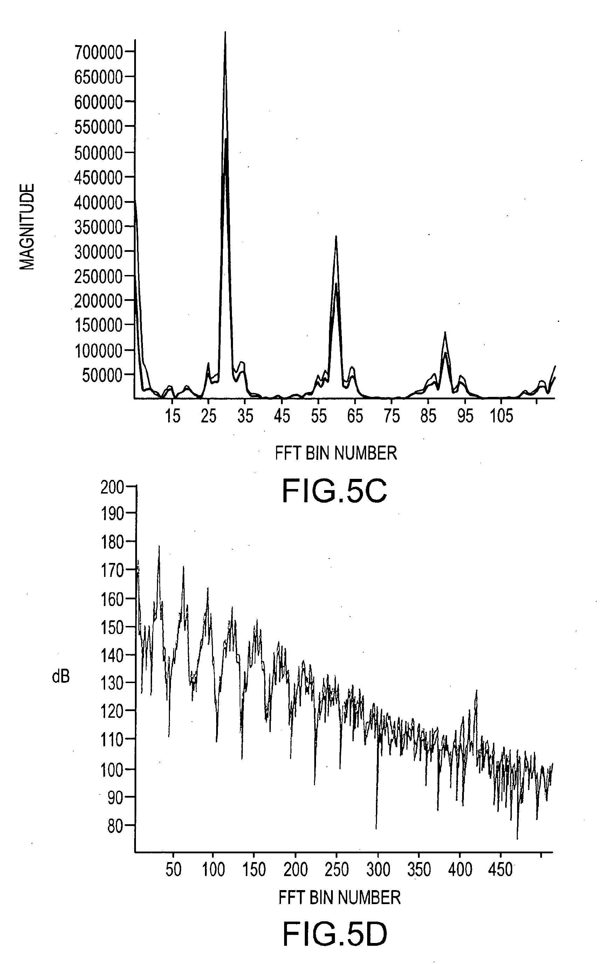

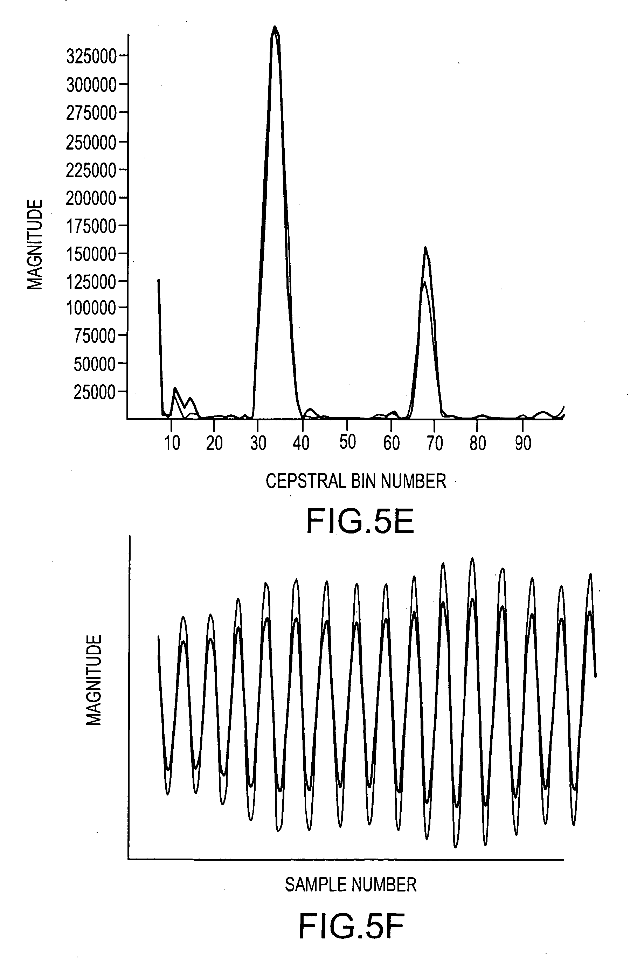

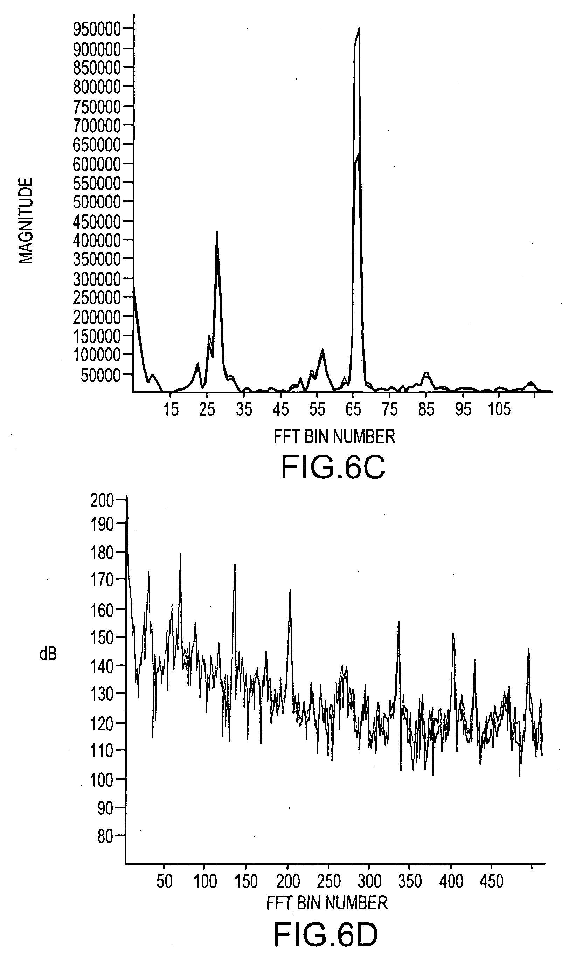

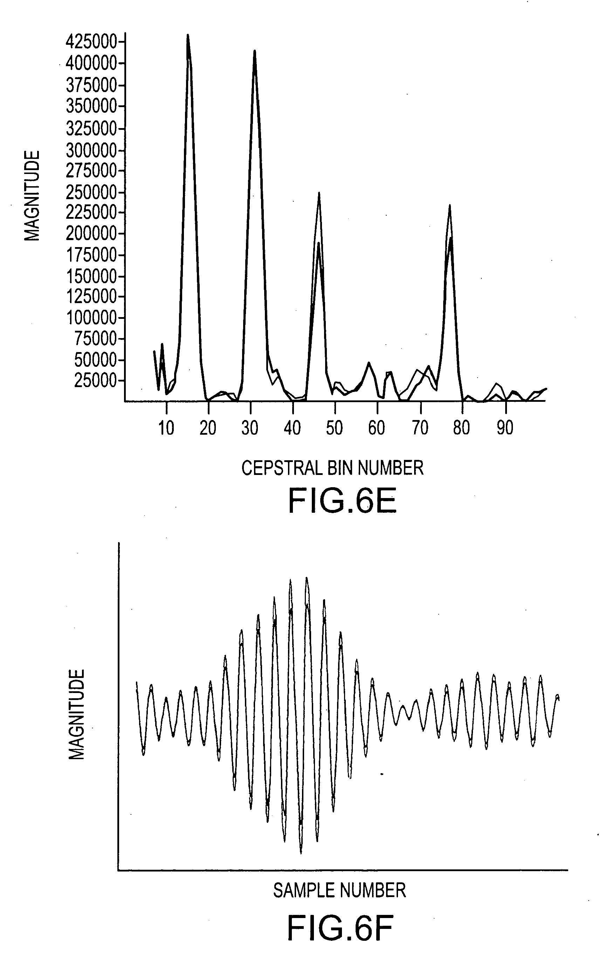

The digitized time domain red and infrared plethysmographic signals are smoothed (420) via a suitable smoothing window (e.g. Hanning, Hamming, or Kaiser) and are then processed in parallel via a complex FFT (430). The output from the first stage FFT is then decoded and the separate red and infrared energy spectra and log power spectra are computed and stored (440, 450). Plots of red and infrared energy spectra and log spectra obtained for the red and infrared signals of FIG. 5A and 6A are shown in FIGS. 5C and 5D, respectively, and in FIGS. 6C and 6D, respectively. A second stage FFT (460) is then applied to the log power spectra to obtain red and infrared cepstra (470) therefrom. If desired, the results of the first and second stage FFT calculations may be scaled to help prevent floating point errors in subsequent computations. Plots of the red and infrared cepstra obtained for the red and infrared signals of FIG. 5A and 6A are shown in FIG. 5E and 6E. Peaks in the cepstra (which has the dimension of Quefrency) are examined (480) and transformed to provide an estimate of pulse frequency. A similar analysis is performed with regard to the spectral signal.

The cepstral based pulse rate estimate is provided to a pulse selection module (490). The pulse selector module (490) also receives estimates of the patient's pulse rate based on examination of peaks in the energy spectra and log power spectra. Additionally, a time-domain pulse rate estimate is extracted (500) from the digitized time domain red and infrared plethysmographic signals via a conventional technique such as differentiation, thresholding and picking the most commonly found interval. Plots of the differentiated waveforms obtained from the time domain red and infrared plethysmographic signals of FIGS. 5A and 6A are shown in FIGS. 5B and 6B. The time domain based pulse rate estimate is also provided as an input to the pulse arbitration module (490).

Information relating to the peaks of the energy spectra and the cepstra are input to a motion classification and motion strength estimation module (510). The motion classification and motion strength estimation module (510) can use one or more of the amplitude, relative position and spacing of the respective peaks in the red and infrared energy spectra and/or cepstra to make motion classification and strength judgments. A simple measure classification and motion estimation can be derived by the number and spacing of cepstral peaks. In this regard, a relatively clean plethysmographic signal will typically produce one major cepstral peak. As the number and size of the cepstral peaks increases, sizable motion components as well as a motion classification can be inferred. Information from the motion classification and motion strength estimation module (510) is input to both an adaptive filter module (520) and the pulse selection module (490).

The adaptive filter module (520) uses estimates of the pulse frequency and the frequency distribution of the motion noise components (if present) to control filtering in the frequency domain in order to improve the signal to noise ratio of the pulse fundamental frequency components and/or its harmonics. In this regard, the red and infrared frequency domain plethysmographic signals obtained after the first stage FFT (430) signals are filtered (530) to produce filtered frequency domain red and infrared plethysmographic signals. Plots of the filtered frequency domain red and infrared plethysmographic signals corresponding to the time domain red and infrared plethysmographic signals of FIGS. 5A and 6A are shown in FIGS. 5F and 6F. A number of different types of filters may be implemented including both finite impulse response (FIR) and infinite impulse response (IIR) filters. One disadvantage of spectral methods is that they are not suited for tracking rapid changes in the input signal. However in the present method (400) the spectral information is used to control an adaptive filter. By using time domain pulse measurement techniques on the output signal from this filter, the ability to track reasonably fast changes is achieved.

An inverse FFT operation (540) is performed to obtain filtered time domain red and infrared plethysmographic signals, and an overlap and add operation (550) is performed to reconstruct the plethysmographic signals minus the DC components and with reduced motion components. Following the overlap and add operation (550), the energy content for both the red and infrared filtered signals is then obtained (560) via, for example, a root-mean-square (rms) measure. This provides an estimate of the AC red and infrared levels. Although not shown in FIG. 4, it is also possible to obtain an estimate of the red and infrared AC levels via the cepstral domain. The main peak location of the red and infrared cepstra can be translated to a frequency value and the value of the energy for that frequency and its harmonics can be obtained (i.e., integrated) by referring to the stored energy spectrum for the red and infrared signals. It is also feasible to use the relative amplitudes of the red and infrared cepstral peaks to derive an AC estimate. Following the overlap and add operation (550), another conventional time domain based pulse estimation is also performed (570) on the filtered red and infrared signals and this estimate is also sent to the pulse arbiter module (490).

The pulse selector (490) selectively uses one of the various time domain, filtered time domain, energy spectra, log power spectra and cepstral based pulse estimates based on the motion strength and classification to provide an overall best estimate (580) of the patient's pulse rate. In this regard, for a range of motions the location of the major cepstral peak suffices as a good estimate of pulse frequency. However for large motion amplitudes and motion that produces waveforms similar to those of red and infrared plethysmographic signals it may be preferred to use other parameters or algorithms. More particularly, the pulse selection module (490) uses the motion estimation and classification derived from the cepstrum in the motion classification and motion strength estimation module (510) to select one of the respective pulse rate estimates. In this regard, different ones of the pulse rate estimates may be indicated for different artifact conditions, based on clinical observations. Under extreme motion conditions, it may not be possible to reliably determine a pulse rate based on current pleth data. In addition to the previously described pulse selection process (490), a neural-net may be employed for enhanced pattern recognition in the pulse selection process (490).

In addition to obtaining an estimate (580) of the patient's pulse rate, the plethysmographic signal processing method (400) of FIG. 4 also derives an estimate of the patient's SPO2 level. The energy content of the time domain red and infrared plethysmographic signals is obtained (590) via, for example a root mean square (rms) transform. This provides an estimate of the red and infrared DC levels. The red and infrared DC levels (590) and AC levels (560) are provided to an SPO2 module (600). As discussed in more detail above in connection with step (310) of the method (200) of FIG. 2, the SPO2 module (600) uses the red and infrared DC and AC levels to derive a measure that can be correlated with the patient's SPO2 level in a manner similar to conventional regression based techniques.

The cepstral domain plethysmographic signal processing method (400) of FIG. 4 also provides for obtaining an enhanced perfusion index (PI) measure when motion artifact is present in the red and infrared time domain plethysmographic signals as compared to known time domain based perfusion index measures. The perfusion index is a measure of relative perfusion in the patient tissue site and is indicative of pulse strength. A time-domain based perfusion index measure may be obtained by, for example, calculating normalized plethysmographic signal amplitudes for the red and infrared time domain plethysmographic signals by summing the normalized delta amplitudes covering the rising portion of one cycle of the pulse waveform. This value can be termed Snda. In this regard, the perfusion index may be calculated from the red and infrared Snda values in accordance with the following expression:

$$PI = (Snda\ (red) * 0.0563 + Snda\ (infrared) * 0.3103) * Scaling\ Factor$$

Further detail regarding such a known time domain based method for obtaining a perfusion index measure is described in United States Patent No. 5,766,127 entitled "METHOD AND APPARATUS FOR IMPROVED PHOTOPLETHYSMOGRAPHIC PERFUSION-INDEX MONITORING", the entire disclosure of which is incorporated herein.

However it is also possible to obtain a measure of the red and infrared plethysmographic signal amplitudes from their respective energy spectrums when the frequency components present in the energy spectrums due to the pulse signal can be identified via processing of the red and infrared cepstrums. In this regard, the plethysmographic signal processing method (400) may incorporate a perfusion index estimator step (610) wherein the red and infrared cepstrums obtained in step (470) are used to identify the frequency components present in the red and infrared energy spectrums obtained in step (440) that are associated with the pulse rate of the patient (i.e. the fundamental pulse frequency and its harmonics). The perfusion index estimator module (610) computes normalized amplitudes for the identified red and infrared spectral peaks. A perfusion index value (620) may then be computed from the normalized amplitudes of the identified red and infrared spectral peaks in accordance with, for example, the following expression:

$$PI = (ESamp\ (red) * 0.0563 + ESamp(infrared) + 0.3103) * ESscaling$$

where ESamp(red) and ESamp(infrared) are the normalized amplitudes derived from the identified spectral peaks in the red and infrared energy spectrums and ESscaling is a scaling factor adjusted to give the spectral PI measure an equivalent value to the time domain PI measure. Because the spectral PI measure uses normalized amplitudes of the identified peaks in the red and infrared spectrums associated with the fundamental pulse frequency, the spectral PI measure is less susceptible to corruption by motion artifact present in the time domain plethysmographic signals since peaks associated with motion artifact will be ignored when identifying the fundamental pulse frequency peaks using the cepstrums.

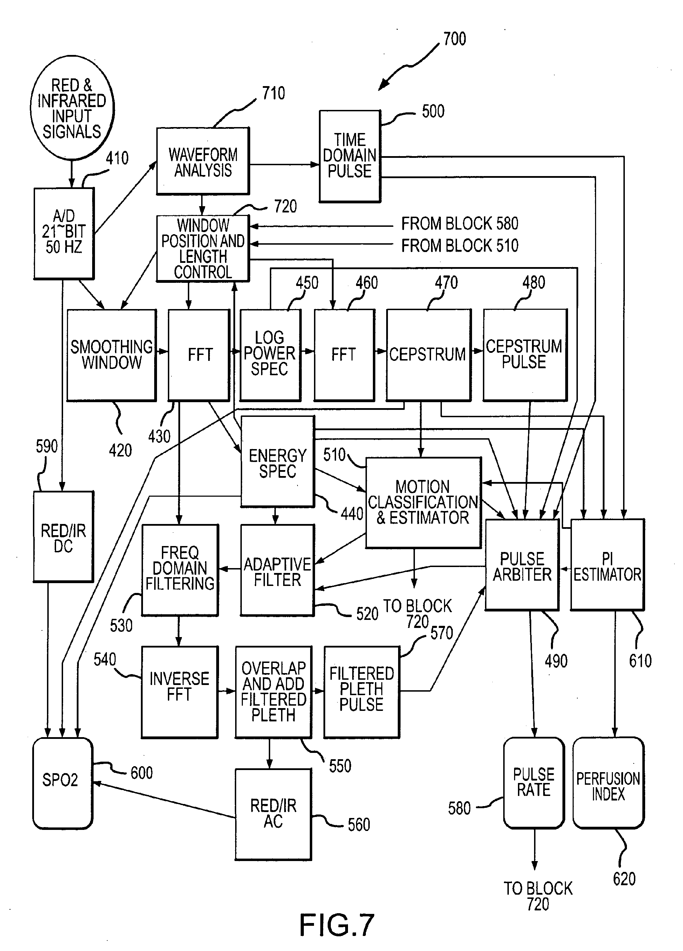

Referring now to FIG. 7, there is shown a block diagram illustrating another implementation of a method (700) for processing the red and infrared time domain plethysmographic signals in multiple domains to obtain desired information relating to patient physiological conditions such as patient pulse rate and blood analyte level (e.g., SPO2) information. The cepstral domain plethysmographic signal processing method (700) shown in FIG. 7 proceeds in a manner similar to the method (400) shown in FIG. 4 and, to the extent that various steps are identical or substantially identical, the same reference numerals are utilized in FIG. 7 as in FIG.4. In addition to the various steps and modules included in the cepstral domain plethysmographic signal processing method (400) of FIG. 4, the multi-domain plethysmographic signal processing method (400) of FIG. 7 includes a waveform analysis module (710) and a window position and length control module (720).

The waveform analysis module (710) is interposed between the step of digitizing (410) the analog red and infrared plethysmographic signals and extracting (500) a time-domain based estimate of the patient's pulse rate. In the waveform analysis module (710), the digitized red and infrared plethysmographic time domain waveforms are analyzed to extract desired information from the waveforms. Extracted information may include time domain features from a differentiated waveform such as spike width and height and variability of these features to identify a region of motion free or motion reduced pulse signal. Such information may also be used for artifact measurement and classification.

The information extracted in the waveform analysis module (710) is provided to the window position and length control module (720). The energy spectra (440) of the FFT transformed red and infrared plethysmographic signals, information from the motion classification and estimation module (510), and the patient's pulse rate (580) is also provided to the window position and length control module (720). The window position and length control module (720) adjusts the position and length of the smoothing window (also referred to in the context of the method of FIG. 7 as a data selection window) applied in the smoothing step (420) and/or the length of the FFT size utilized in the first and second stage FFT steps (430, 460). Under the direction of the window position and length control module (720), the length of the smoothing window and/or the FFT size may be shortened or lengthened as necessary in order to optimize extraction of plethysmographic signal components relating to patient physiological conditions (e.g., pulse rate, SPO2 level) from noise components that may also be present in the plethysmographic signals. In this regard, for patients having typically higher pulse rates (e.g., babies and neonates) smaller window lengths and shorter FFT sizes have been found to be appropriate while for patients with typically slower pulse rates (e.g., adults) longer window lengths and larger FFT sizes have been found to provide more optimal results.

In addition to controlling window length and FFT size, the window position and length control module (720) also controls the position of the smoothing window. In this regard, when motion artifact is present in the red and infrared plethysmographic signals, signal regions having little or no motion artifact present may be identified (e.g., by the motion classification and estimation module (510)) and a window (with its length adjusted as appropriate to select the low-noise regions) can then be selectively positioned over such regions for subsequent spectral and cepstral processing. In this regard, a two-pass system may be implemented wherein the plethysmographic signals are initially processed without using a window to identify signal portions that are free from motion or include only limited motion, and then are re-processed using a window that is appropriately positioned and adjusted to select only the identified no or low noise regions.

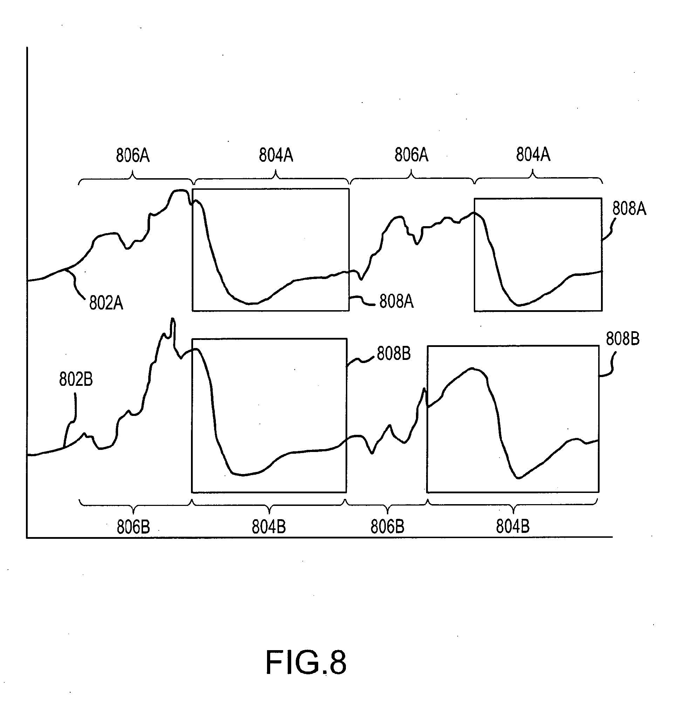

By way of example, FIG. 8 shows plots of exemplary red and infrared plethysmographic signals 802A, 802B that include no or low motion regions 804A, 804B and high or severe motion regions 806A, 806B. Under the direction and control of the window position and length control module (720) several data selection windows 808A, 808B, are positioned and have their length appropriately adjusted to select only the no or low noise regions 804A, 804B of the red and infrared plethysmographic signals for further processing.

In addition to obtaining a measure correlated with the patients SPO2 level from the red and infrared DC and AC levels, the SPO2 module (600) of the multi-domain plethysmographic signal processing method (700) of FIG. 7 also derives an SPO2 measure from the red and infrared energy spectrums (440) and the red and infrared cepstrums (470). In this regard, the SPO2 module (600) may compare the energy present around the fundamental component of the red energy spectrum to the energy present around the fundamental component of the infrared energy spectrum to derive a ratio that is correlated with the patient's SPO2 level. Similarly, information present in the red and infrared cepstrums may be used by the SPO2 module (600) to derive a ratio that is correlated with the patient's SPO2 level.

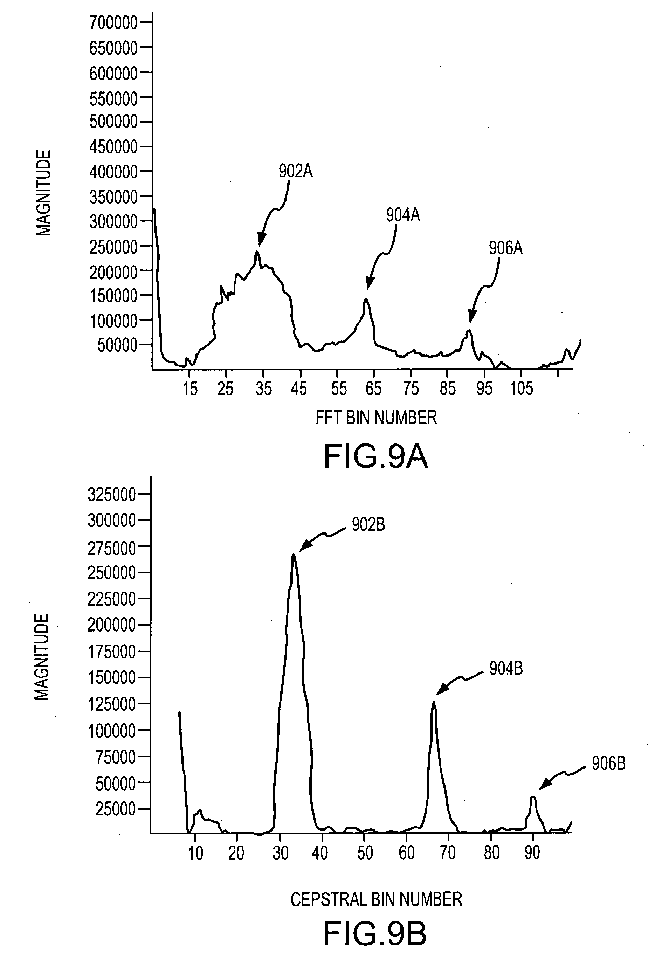

Referring now to FIGS. 9A-9B, in the multi-domain signal processing methods (200, 400, 700) of FIGS. 2, 4 and 7, the time, spectral and cepstral domains are analyzed and evaluated, and features identified in one domain may be confirmed and correlated in the other domains. For example, as illustrated in FIGS. 9A-9B, the fundamental spectral component 902A of one of the plethysmographic signals may be obscured by motion artifact and other noise. This can make it difficult to obtain the SpO2 level of the patient from the energy spectrum by comparing spectra for the red and infrared signals. However, prominent cepstral peaks 902B, 904B, 906B can be used to search for related spectral components 902A, 904A, 906A since a cepstral component can more easily be identified even though the region around the fundamental spectral component 902A may be corrupted by noise or motion components. Once identified in the cepstral domain, the SPO2 content may be extracted by the SPO2 module (600) directly from the energy spectrum by processing the second or third harmonic regions 904A, 906A, which may be distant enough from the lower frequency noise, since the SPO2 energy related content of the harmonic spectral components 904A, 906A is typically similar to that of the fundamental spectral component 902A. In this regard, such technique may be described as cepstral identification of fundamental spectral components and related harmonics followed by SPO2 evaluation at multiple harmonic sites.

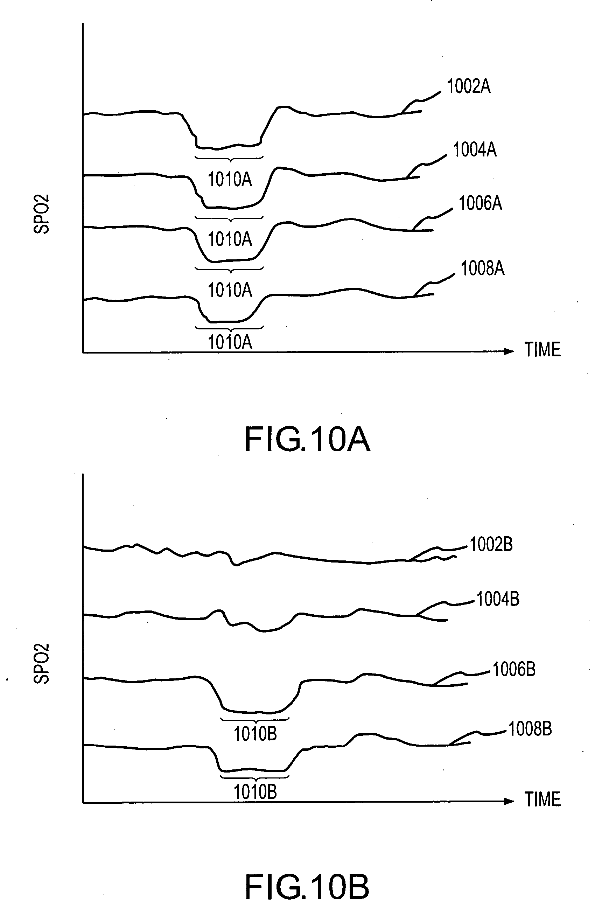

Referring now to FIGS. 10A-10B, the SPO2 module (600) may confirm the accuracy of the time domain, spectral domain and cepstral domain based SPO2 level estimates through a technique referred to herein as "sheparding" the estimates. The sheparding technique recognizes that while a direct current (DC) tracking based SPO2 value typically does not accurately represent the correct magnitude of the patient's SPO2 level, the shape of the DC tracking based SPO2 plot is typically correct over time. Thus, the time domain, spectral domain and cepstral domain based SPO2 levels determined in the SPO2 module (600) may be plotted over time and the shape of the plots compared with a plot of a DC tracking based SPO2 value also determined in the SPO2 module (600). Through such comparison, the accuracy of the various SPO2 estimates may be confirmed and, if one or more of the estimates does not appear to be accurate, such SPO2 value can be rejected and only the accurate values reported and/or further utilized by the SPO2 module (600).

By way of example, FIG. 10A shows plots of exemplary time domain 1002A, spectral domain 1004A, cepstral domain 1006A, and DC tracking 1008A based SPO2 levels wherein the shape of each of the plots is similar. In this regard, each plot 1002A-1008A includes a corresponding shallow dip or desaturation region 1010A wherein the SPO2 level of the patient drops for a period of time and then recovers. Since, the desaturation region 1010A appears in each plot 1002A-1008A at substantially the same time, ends at substantially the same time, and has substantially the same shape, all three of the filtered time domain, spectral domain and cepstral domain based SPO2 estimates 1002A-1006A appear to be accurate and provide confirmation of the accuracy of the other estimates.

By way of further example, FIG. 10B shows plots of exemplary time domain 1002B, spectral domain 1004B, cepstral domain 1006B, and DC tracking 1008B based SPO2 levels wherein the shape of each of the plots is not similar due, for example, to the presence of motion artifact in the original red and infrared plethysmographic signals. In this regard, the DC tracking based SPO2 plot 1008B includes a desaturation region 1010B which also appears distinctly in the cepstral domain based SPO2 plot 1006B but does not distinctly appear in the time domain and spectral domain based plots 1002B, 1004B. Thus, the accuracy of the time domain and spectral domain based SPO2 levels during the period of time covered by the desaturation region 1010B is questionable and the cepstral domain based SPO2 estimate appears to be accurate.

As may be appreciated, during certain time periods, none of the time domain, spectral domain and cepstral domain based SPO2 estimates may accurately follow the shape of the DC tracking based SPO2 estimate, in which case all three may be rejected by the SPO2 module (600). During such instances, the SPO2 module (600) may, for example, report an earlier SPO2 value previously confirmed to be accurate, or it may report an appropriately scaled DC tracking based SPO2 estimate.

During periods when all three of the filtered time domain, spectral domain and cepstral domain based SPO2 tracks agree in form with the DC track and with each other (such as illustrated in FIG. 10A), it can be assumed that the AC information included in the filtered time domain, spectral domain and cepstral domain SPO2 tracks is good or is at least being accurately extracted in motion conditions. At such times, the SPO2 values from the three tracks can be used to calibrate the DC SPO2 track and thereby generate a second DC SPO2 track that agrees both in form and in value with the previous SPO2 values. The second (calibrated) DC SPO2 track (and parameters describing the track) may be used to predict SPO2 values during periods of severe motion when none of the filtered time domain, spectral domain, or cepstral domain SPO2 tracks agrees in form with the non-calibrated DC SPO2 track. In order to generate the second (calibrated) DC SPO2 track during appropriate periods and to properly utilize the second (calibrated) DC SPO2 track during periods of severe motion, it may be necessary to maintain a history of the various SPO2 values and motion estimates.

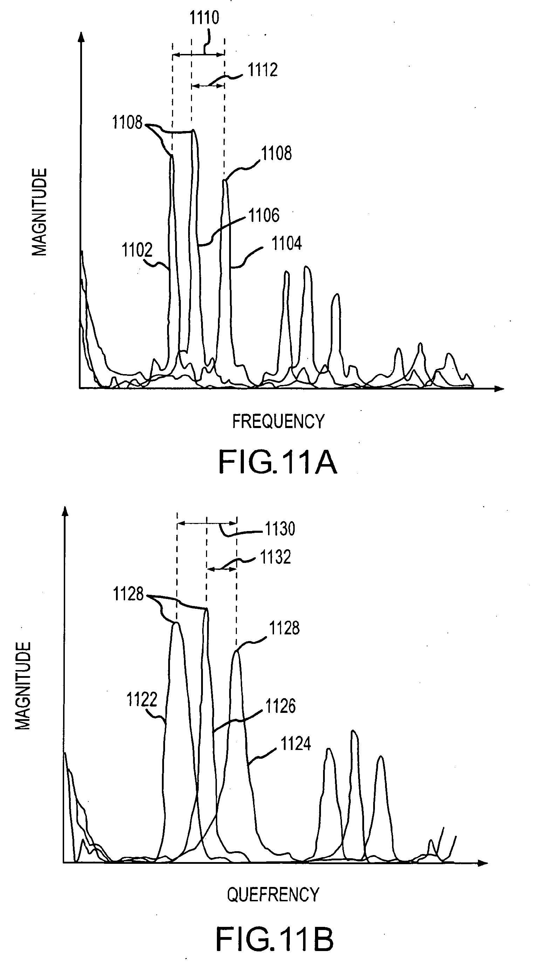

Referring now to FIGS. 11A-B, the motion classification and strength estimation module (510) may analyze the red and infrared spectrums and cepstrums in a number of manners in order to identify the presence of motion artifact in the red and infrared plethysmographic signals. One manner is to compare successive frames or snapshots of the spectrums and cepstrums over time to determine if there is jitter present in the peaks of the spectrums and cepstrums.

By way of example, FIG. 11A shows three successive frames of exemplary infrared spectrums 1102, 1104, 1106. As can be seen in FIG. 11A, over time the fundamental spectral peak 1108 (and its related harmonic components) drifts from a lower frequency to a higher frequency and back to a lower frequency again. By measuring the amount of frequency drift of the spectral peak 1108 and comparing the measured drift to one or more threshold values, it is possible to classify the strength of any motion present in the plethysmographic signals. For example, the absolute value of the frequency drift 1110 measured between the spectral peak 1108 of the first instance of the spectrum 1102 and the spectral peak 1108 of the second instance of the spectrum 1104 may exceed a higher threshold value thereby indicating the presence of severe motion during the time between the first and second instances of the spectrum 1102, 1104, whereas the absolute value of the frequency drift 1112 measured between the spectral peak 1108 of the second instance of the spectrum 1104 and the spectral peak 1108 of the third instance of the spectrum 1106 may exceed a lower threshold value but not the higher threshold value thereby indicating the presence of clinical motion during the time between the second and third instances of the spectrums 1104, 1106. As may be appreciated, where the measured frequency drift is below the lower threshold value, the plethysmographic signal may be classified as having no or only insignificant motion during the time period between successive spectral frames.

Likewise, FIG. 11B shows three successive frames of exemplary infrared cepstrums 1122, 1124, 1126. As can be seen in FIG. 11B, over time the primary cepstral peak 1128 corresponding with the fundamental spectral peak (and the smaller cepstral peaks corresponding to the harmonic spectral components) drifts from a lower Quefrency to a higher Quefrency and back to a lower Quefrency again. By measuring the amount of Quefrency drift and comparing the measured drift to one or more threshold values, it is possible to classify any motion present in the plethysmographic signals as well as obtain an indication of a magnitude of such motion. For example, the absolute value of the Quefrency drift 1130 measured between the cepstral peak 1128 of the first instance of the cepstrum 1122 and the cepstral peak 1128 of the second instance of the cepstrum 1124 may exceed a higher threshold value thereby indicating the presence of severe motion during the time between the first and second instances of the cepstrum 1122, 1124, whereas the absolute value of the Quefrency drift 1132 measured between the cepstral peak 1128 of the second instance of the cepstrum 1124 and the cepstral peak 1128 of the third instance of the cepstrum 1126 may exceed a lower threshold value but not the higher threshold value thereby indicating the presence of clinical motion during the time between the first and second instances of the cepstrums 1122, 1128. As may be appreciated, where the measured Quefrency drift is below the lower threshold value, the plethysmographic signal may be classified as having no or only insignificant motion during the time period between successive cepstral frames.

As noted above, it is desirable to distinguish useful physiological information from interfering information. This can be done for a variety of purposes including selectively filtering the interfering information from the useful information and selecting a preferred processing technique. In the context of the system described above, where different processing techniques are utilized depending on the motion or noise environment, useful physiological information may be distinguished from interfering information in order to select a processing technique. For example, in the case of a relatively clean signal, the signal may be filtered using a bandpass filter tuned to the pulsatile frequency and the resulting filtered signal may be utilized to calculate blood oxygen saturation. This is an AC component algorithm. In other cases, DC tracking may be utilized together with a previously calculated value of blood oxygen saturation in order to calculate an estimate of the current blood oxygen saturation value.

It has been noted that distinguishing useful physiological information from interfering information based on a shape or waveform of the spectrum or spectra can be problematic. In particular, such analyses can result in failing to perform an AC analysis in certain cases such as a rapidly changing pulse rate or an arrhythmia. It will be appreciated that, although a relatively clean signal may be obtained in such cases, a spectral analysis of the signal may be inconclusive or suggest the presence of a strong interfering signal. In addition, in certain cases a spectral analysis may indicate that the signal is acceptable when, in fact, significant interference is present and mimics a physiological signal in the spectral domain.

These problems can be more fully addressed in accordance with the present invention by monitoring certain parameters that are substantially independent of the shape or waveform of the spectrum. Two such parameters described below are the observed energy ratio of the red and infrared fundamental spectral peaks ("observed fundamental energy ratio") and the phase of the first harmonic in relation to the fundamental ("relative phase"). Each of these is described in turn below.

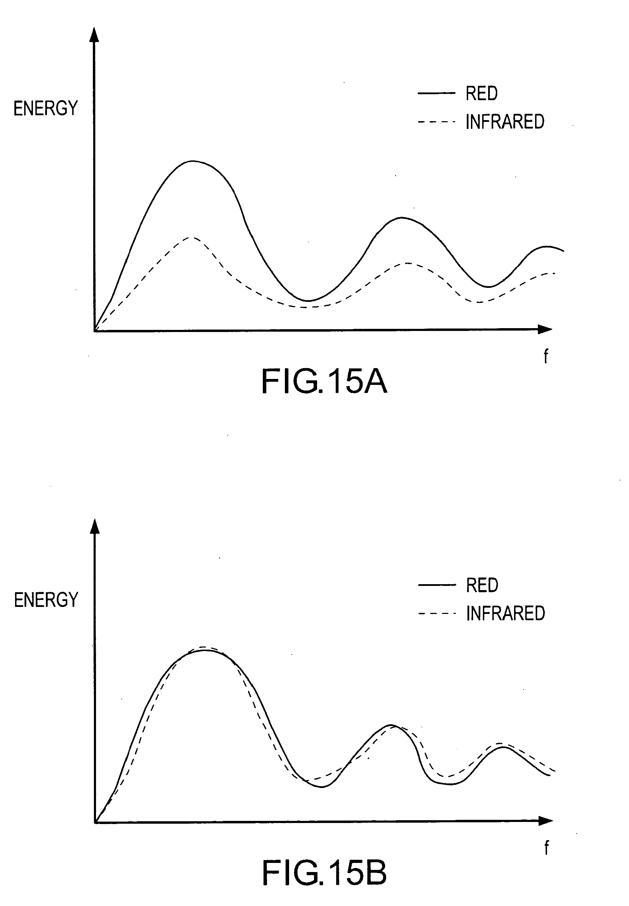

Referring to Figs. 15A and 15B, exemplary and somewhat idealized (for purposes of illustration) spectra for relatively clean detector signals and artifact affected signals, respectively, are shown. As shown in 15A, in the case of a relatively clean signal, the red and infrared spectra generally appear well correlated but with different peak energies. Indeed, it has been found that, in the case of clean signals, the ratio of the peak energy of the fundamental peak of the red spectrum to the peak energy of the fundamental peak of the infrared spectrum remains substantially constant, at least over relatively short periods of time.

By contrast, artifact affected signals, as shown in Fig. 15B, generally yield different spectra. In many cases, as depicted in Fig. 15B, the energy of the fundamental peak of an artifact affected signal tends to be about the same in the red and infrared spectra.

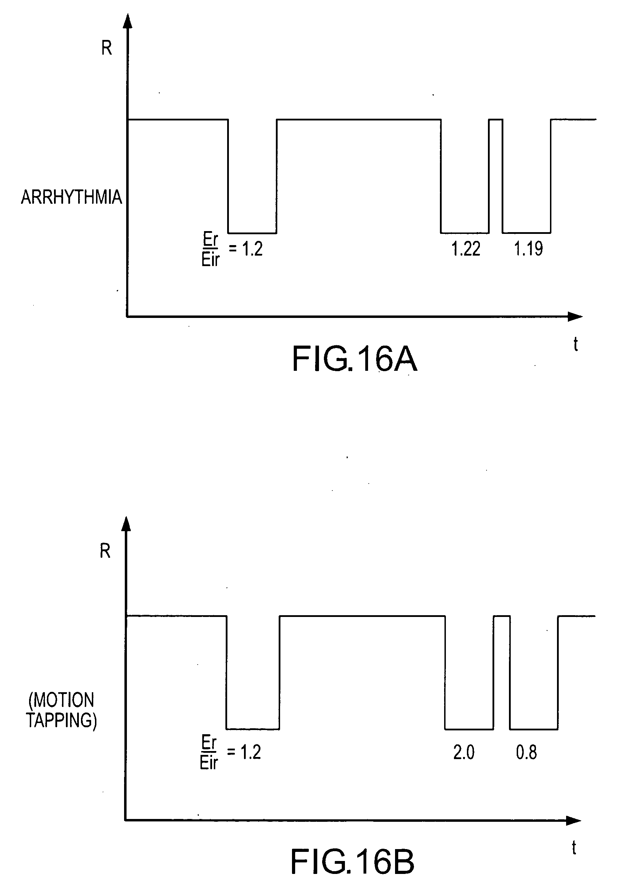

Figs. 16A and 16B illustrate perceived variations and R-ratio or related values associated with arrhythmia and periodic motion examples, respectively. As shown, each of the curves includes a number of events where the R-ratio is perceived to drop suddenly or "bucket." Such bucketing may be due to artifact such as patient motion. Because patient motion tends to have similar effects on the red and infrared signals, such motion events tend to draw the measured R-ratio, in the absence of any corrective algorithm, towards one, corresponding to a blood oxygen saturation of about 82 or 83 percent. As shown, a similar phenomenon may be observed in the case of arrhythmia due to a failure to accurately track the pulsatile signal. It would be desirable to distinguish motion effect such as illustrated in Fig. 16B from arrhythmia effects as illustrated in Fig. 16A, as it may be possible to obtain good oxygen saturation information in the case of arrhythmia.

It has been found that the observed fundamental energy ratio remains substantially constant in bucketing events associated with arrhythmia, whereas this ratio fluctuates significantly in the case of motion or other artifact. Accordingly, good signals can be reliably distinguished from artifact affected signals by tracking the observed fundamental energy ratio over time. Specifically, by defining an appropriate variability threshold, signals that evidence stability of this ratio can be accepted or validated for appropriate processing, e.g., for AC analysis to determine a value of blood oxygen saturation, whereas less stable signals can be invalidated, e.g., resulting in DC tracking processing. This is illustrated in Figs. 16A and 16B where successive bucketing events in the case of arrhythmia are associated with observed fundamental energy ratios of 1.20, 1.22 and 1.19 whereas successive bucketing events in the motion example of Fig.

16B correspond to observed fundamental energy ratios of 1.2, 2.0 and 0.8.

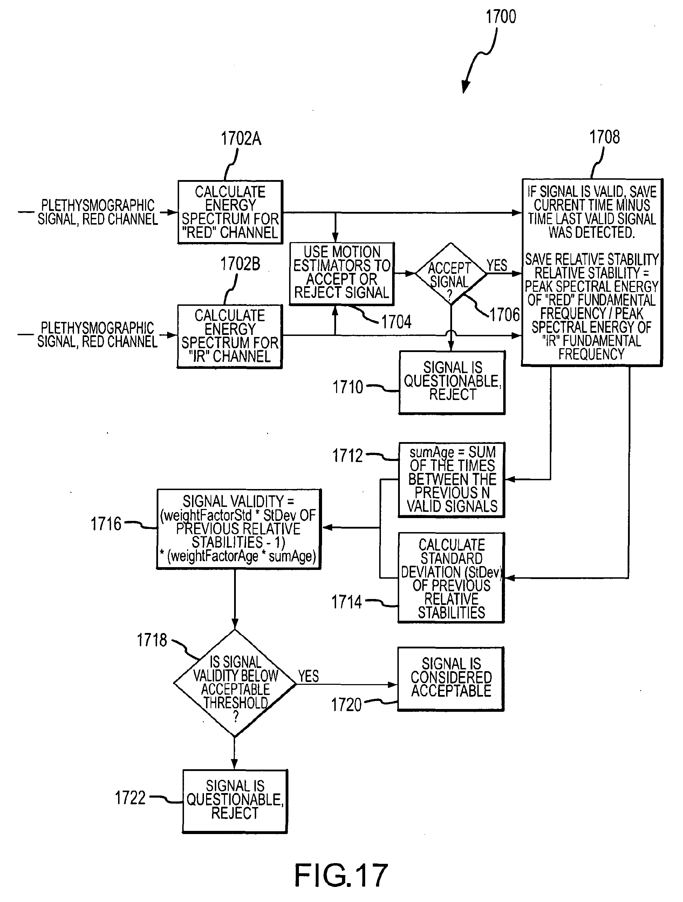

An associated processing flowchart is illustrated in Fig. 17. The illustrated process 1700 is initiated by calculating (1702A and 1702B) the energy spectra for the red and infrared channels. This generally involves performing a Fast Fourier transform on the time-based detector signal information and then computing power spectra in conventional fashion. Motion estimators are then used (1704) to accept or reject signals. As noted above, various peak qualifications and related parameters may be utilized in this regard to distinguish apparently clean signals from apparently motion affected signals. Based on this analysis, a decision is made (1706) to accept or reject the signal. If the signal is questionable, it is rejected (1710). If the signal is acceptable based on these criteria, a time parameter is calculated and a relative stability is saved (1708). In this regard, the time parameter can be calculated as the current time minus the time the last valid signal was detected. The relative stability is calculated as the peak spectral energy of the red fundamental frequency divided by the peak spectral energy of the infrared fundamental frequency.

A SumAge parameter is then calculated (1712) as the sum of the times between the previous end valid signals. Additionally, a standard deviation is calculated (1714) of previous relative stabilities. The standard deviation in this regard can be calculated by the following formula:

$$\text{stdev of previous n relative stabilities} = \sqrt{\frac{n\sum x^2 - \left(\sum x\right)^2}{n(n-1)}}$$

where n = number of relative stability measurements
where x = specific relative stability measurement as defined by $(R_n/IR_n - R_{n-1}/IR_{n-1})$
where R = peak spectral domain value
where IR = peak infrared spectral domain value

These two factors are used to determine (1716) signal validity. Specifically, the signal validity is calculated by the following formula:

$$\text{Validity} = (\text{weightFactorstd} * \text{stdev previous n relative stabilities}) - 1) * \text{weightFactorAge} + \text{Summation previous n valid times}$$

The empirically derived wavefactorstd and weightfactorage may be set so as to yield desired results. In this regard, it may be desired to exclude significant artifact affected signals while allowing for varying observed fundamental energy ratios associated with true desaturation events. In this regard, a weightfactorstd of 20 and a weightfactorage of 25 have been found to provide good results. A determination is then made (1718) whether the signal validity is below an acceptable threshold. Again, this threshold may be set empirically. If the signal is questionable, it is rejected (1722). Otherwise the signal is considered (1720) acceptable. As noted above, acceptable signals may be processed using an AC analysis algorithm to determine the current value of SpO$_2$.

Another parameter which may be utilized to distinguish good signals from artifact affected signals is the relative phase. In this regard, it has been observed that the relative phase between the fundamental of the pulsatile signal and the first harmonic remains within a certain range for most patients in most situations. This is reflected in the observed plethysmographic spectra for patients where the dichrotic notch has a defined range of positions in relation to a pulsatile waveform peak. That is, the relative phase of the first harmonic is correlated to the position of the dichrotic notch.

Moreover, it has been observed that the spectra associated with artifact affected signals do not follow this pattern. In particular, the relative phase of the first harmonic can vary across a range of phase values.

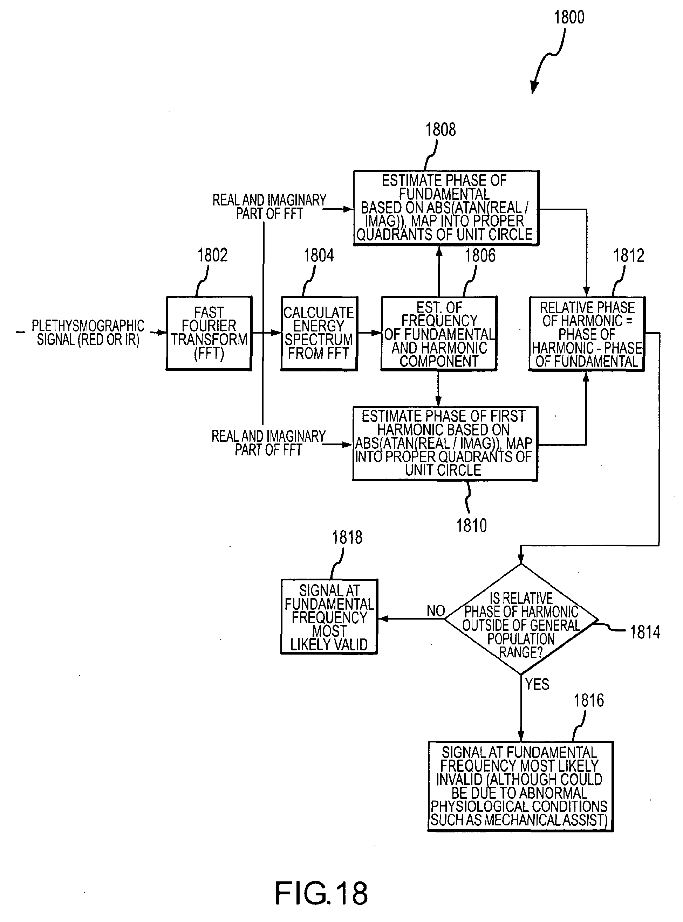

This relative phase phenomenon can be used to distinguish good signals from artifact affected signals. Specifically, this phenomenon can be used alone or together with other indicators as discussed above to distinguish good signals from artifact affected signals, e.g., to validate signals for AC analysis. An associated process 1800 is illustrated in Fig. 18. The process 1800 is initiated by performing (1802) a Fast Fourier Transform on the red or infrared photophethysmographic signal. An energy spectrum is then calculated (1804) from the Fast Fourier Transform. It should be noted in this regard that the imaginary component of the Fast Fourier Transform function is needed to calculate phase related information. This imaginary component is lost in calculating the energy spectrum. Accordingly, the raw Fast Fourier Transform information is provided to modules 1808 and 1810 as described below.

The frequency of the fundamental and harmonic components can then be estimated (1806) from the energy spectrum. In particular, as noted above, certain peak qualification parameters are applied to the spectral peaks to identify potential fundamental and harmonic peaks. A potential fundamental peak can then be matched to a potential harmonic peak to identify the apparent fundamental and harmonic of the pulsatile signal.

The phase of the fundamental is then estimated (1808) based on the following formula:

$$\Theta = \text{abs}(a\tan(\text{real}/\text{imag}))$$

This phase is mapped into the proper quadrant of a unit circle to provide a useful phase value. Similarly, the phase of the first harmonic is estimated using the same process for the first harmonic component. The relative phase of the harmonic is then calculated (1812) as the phase of the harmonic minus the phase of the fundamental. A determination is then made (1814) whether this relative phase is outside of the general population range. If it is, the signal at the fundamental frequency is deemed to be invalid (1816). It will be appreciated that in some cases, such as mechanical heart-assist situations, clean signal information may be lost in this regard. If the relative phase is outside of the general population range, the signal is determined to be valid, e.g., thereby enabling an AC algorithm for calculating blood oxygen saturation. Whether the relative phase is inside or outside of this range can be determined, for example, based on comparison to static thresholds derived empirically based on analysis of patient data or can be developed heuristically for a given patient or based on many patients. It will be appreciated that phase information may be used for a variety of other purposes, such as providing a better waveform including proper positioning of the dichrotic notch in the case of waveforms generated from signal information filtered using an adaptive filter tuned to the fundamental frequency of the pulsatile signal.

While various embodiments of the present invention have been described in detail, further modifications and adaptations of the invention may occur to those skilled in the art. However, it is to be expressly understood that such modifications and adaptations are within the spirit and scope of the present invention.

**Claims**

1. A method for use in a pulse oximetry system, comprising the steps of:

   obtaining a time-based signal representative of an optical signal potentially including physiological information based on interaction with a patient;
   first performing a transform on said time-based signal to obtain frequency related information;
   second performing an analysis on said frequency related information to obtain a value independent of a shape and waveform of a spectrum of said time-based signal; and
   using said value to determine a quality of said time-based signal in relation to one of physiological information content and artifact content.

2. A method as set forth in Claim 1, wherein said step of first performing comprises obtaining first frequency related information corresponding to a first portion of said time-based signal, obtaining second frequency related information corresponding to a second portion of said time-based signal, and using said first and second frequency related information to calculate said value.

3. A method as set forth in Claim 2, wherein said step of obtaining comprises receiving first channel information corresponding to a first channel of said optical signal having a first spectral content and second channel information corresponding to a second channel of said optical signal having a second spectral content different than said first spectral content.

4. A method as set forth in Claim 3, wherein said first frequency related information corresponds to said first channel information and second frequency related information corresponds to said second channel information.

5. A method as set forth in Claim 3, wherein said step of second performing comprises calculating a parameter indicative of a relative energy of said first and second channels.

6. A method as set forth in Claim 5, wherein said relative energy relates a peak energy of the first channel and a peak energy of the second channel.

7. A method as set forth in Claim 5, wherein said relative energy involves a ratio of a peak energy of the first channel to a peak energy of the second channel.

8. A method as set forth in Claim 5, further comprising the step of monitoring said parameter over time.

9. A method as set forth in Claim 2, wherein said first frequency related information corresponds to a fundamental component of said time-based signal and said second frequency related information corresponds to a harmonic component of said time-based signal.

10. A method as set forth in Claim 9, wherein said step of second performing comprises calculating a relative phase of said harmonic component in relation to said fundamental component.

11. A method as set forth in Claim 1, further comprising the step of determining blood oxygen saturation information.

12. A method as set forth in Claim 1, further comprising the step of determining pulse rate information.

13. A method as set forth in Claim 10, further comprising the step of displaying a plethysmographic waveform when said physiological information is obtained, wherein said step of displaying comprises using information corresponding to said phase of said harmonic component.

14. An apparatus for use in a pulse oximetry system, comprising:

a port for receiving a time-based representative of an optical signal potentially including physiological information based on interaction with a patient; and
a processor operative for performing a transform on said time-based signal to obtain frequency related information, performing an analysis on said frequency related information to obtain a value independent of a shape and waveform of a spectrum of said time-based signal, and using said value to determine a quality of said time-based signal in relation to one of physiological information content and artifact content.

15. An apparatus as set forth in Claim 1, wherein said processor is operated for obtaining first frequency related information corresponding to a first portion of said time-based signal, obtaining second frequency related information corresponding to a second portion of said time-based signal and using said first and second frequency related information to calculate said value.

16. An apparatus as set forth in Claim 15, wherein said processor is operative for receiving first channel information corresponding to a first channel of said optical signal having a first spectral content and second channel information corresponding to a second channel of said optical signal having a second spectral content different than said first spectral content.

17. An apparatus as set forth in Claim 16, wherein said first frequency related information corresponds to said first channel information and second frequency related information corresponds to said second channel information.

18. An apparatus as set forth in Claim 16, wherein said processor is operative for calculating a parameter indicative of a relative energy of said first and second channels.

19. An apparatus as set forth in Claim 18, wherein said relative energy relates a peak energy of the first channel and a peak energy of the second channel.

20. An apparatus as set forth in Claim 18, wherein said relative energy involves a ratio of a peak energy of the first channel to a peak energy of the second channel.

21. An apparatus as set forth in Claim 18, wherein said processor is operative for monitoring said parameter over time.

22. An apparatus as set forth in Claim 15, wherein said first frequency related information corresponds to a fundamental component of said time-based signal and said second frequency related information corresponds to a harmonic component of said time-based signal.

23. An apparatus as set forth in Claim 22, wherein said processor is operative for calculating a relative phase of said harmonic component in relation to said fundamental component.

24. An apparatus as set forth in Claim 14, wherein said processor is operative for determining blood oxygen saturation information.

**25.** An apparatus as set forth in Claim 14, wherein said processor is operative for determining pulse rate information.

**26.** An apparatus as set forth in Claim 23, further comprising a display for displaying a plethysmographic waveform when said physiological information is obtained, wherein said display is based on information corresponding to said phase of said harmonic component.

FIG.1

FIG.2

FIG.3A

FIG.3B

FFT BIN NUMBER

# FIG.3C

FIG.3D

FFT BIN NUMBER

# FIG.3E

FIG.4

FIG.5A

FIG.5B

FFT BIN NUMBER

## FIG.5C

FFT BIN NUMBER

## FIG.5D

EP 1 611 847 A1

FIG.5E

SAMPLE NUMBER

FIG.5F

30

SAMPLE NUMBER

FIG.6A

SAMPLE NUMBER

FIG.6B

FIG.6C

FIG.6D

CEPSTRAL BIN NUMBER

FIG.6E

SAMPLE NUMBER

FIG.6F

FIG.7

FIG.8

FIG.9A

FIG.9B

FIG.10A

FIG.10B

FIG.11A

FIG.11B

1200

START

RECEIVE
DETECTOR SIGNAL — 1202

FILTER/
CONDITION SIGNAL — 1204

DIGITIZE AND
TIME PULSE
DEMODULATE    OR    DIGITIZE AND
FREQUENCY PULSE
DEMODULATE    OR    DIGITIZE AND
CODE PULSE
DEMODULATE

1206A    1206B    1206C

PROCESS CHANNEL
SIGNALS TO
IDENTIFY ARTIFACT
CHARACTERISTICS — 1208

CLASSIFY ARTIFACT — 1210

SELECT ARTIFACT
PROCESSOR BASED
ON ARTIFACT
CLASSIFICATION — 1212

USE ARTIFACT
PROCESSOR 1    OR    USE ARTIFACT
PROCESSOR 2    OR    USE ARTIFACT
PROCESSOR N

1214A    1214B    1214C

CALCULATE $SpO_2$,
PULSE RATE AND
PERFUSION INDEX    CALCULATE $SpO_2$,
PULSE RATE AND
PERFUSION INDEX    CALCULATE $SpO_2$,
PULSE RATE AND
PERFUSION INDEX

1216A    1216B    1216C

FIG.12

FIG.13

FIG.14

1400

1402 — SENSOR — LEDS AND DETECTOR

FAST A/D — IMMEDIATE CONVERSION "ANALOG" TO "DIGITAL" — 1404

PURE DIGITAL WAVEFORM — 52,000 pts/sec — 1406

DEMOD — FREQUENCY AND CODE DEMODULATION — 1408

RDC / RAC IRDC / IRAC — 1410

LOW PERFUSION CIRCUITRY — 1409

TIME DOMAIN — REAL TIME PROCESSING — 1412A

FREQ DOMAIN — POWER SPECTRAL PROCESSING — 1412B

QUEFRENCY DOMAIN — CEPSTRAL PROCESSING — 1412C

MOTION ESTIMATION ENGINE — CLINICALLY BASED DECISION MAKER — 1414

1416A

ADAPTIVE FILTERS — PLETH AND PULSE RATE "CLEANERS" WEIGHTED AVERAGING — 1418

1416B

FILTER CONTROL

FINAL SpO2 CALCULATION & COMPONENT CORRECTION — (TruTrak+ PROCESSING IF NEEDED) — 1420

AVERAGING POST-PROCESSOR — (FROM OLDER TruTrak) — 1422

SATURATION DISPLAY — 1424

FIG.15A

FIG.15B

R

ARRHYTHMIA

$\dfrac{Er}{Eir}$ = 1.2          1.22      1.19

t

FIG.16A

R

(MOTION
TAPPING)

$\dfrac{Er}{Eir}$ = 1.2          2.0      0.8

t

FIG.16B

EP 1 611 847 A1

1700

1702A

PLETHYSMOGRAPHIC SIGNAL, RED CHANNEL → CALCULATE ENERGY SPECTRUM FOR "RED" CHANNEL

1702B

PLETHYSMOGRAPHIC SIGNAL, RED CHANNEL → CALCULATE ENERGY SPECTRUM FOR "IR" CHANNEL

USE MOTION ESTIMATORS TO ACCEPT OR REJECT SIGNAL
1704

ACCEPT SIGNAL ?
1706

YES

1708

IF SIGNAL IS VALID, SAVE CURRENT TIME MINUS TIME LAST VALID SIGNAL WAS DETECTED.

SAVE RELATIVE STABILITY RELATIVE STABILITY = PEAK SPECTRAL ENERGY OF "RED" FUNDAMENTAL FREQUENCY / PEAK SPECTRAL ENERGY OF "IR" FUNDAMENTAL FREQUENCY

1710 — SIGNAL IS QUESTIONABLE, REJECT

1712 — sumAge = SUM OF THE TIMES BETWEEN THE PREVIOUS N VALID SIGNALS

1716 — SIGNAL VALIDITY = (weightFactorStd * StDev OF PREVIOUS RELATIVE STABILITIES - 1) * (weightFactorAge * sumAge)

CALCULATE STANDARD DEVIATION (StDev) OF PREVIOUS RELATIVE STABILITIES
1714

1718 — IS SIGNAL VALIDITY BELOW ACCEPTABLE THRESHOLD ?

YES

SIGNAL IS CONSIDERED ACCEPTABLE
1720

1722 — SIGNAL IS QUESTIONABLE, REJECT

FIG.17

1800

1808

REAL AND IMAGINARY
PART OF FFT

ESTIMATE PHASE OF
FUNDAMENTAL
BASED ON ABS(ATAN(REAL /
IMAG)), MAP INTO PROPER
QUADRANTS OF UNIT CIRCLE

1802    1804    1806    1812

PLETHYSMOGRAPHIC
SIGNAL (RED OR IR)

FAST
FOURIER
TRANSFORM
(FFT)

CALCULATE
ENERGY
SPECTRUM
FROM FFT

EST. OF
FREQUENCY
OF FUNDAMENTAL
AND HARMONIC
COMPONENT

RELATIVE PHASE
OF HARMONIC =
PHASE OF
HARMONIC - PHASE
OF FUNDAMENTAL

REAL AND IMAGINARY
PART OF FFT

ESTIMATE PHASE OF FIRST
HARMONIC BASED ON
ABS(ATAN(REAL / IMAG)), MAP
INTO PROPER QUADRANTS OF
UNIT CIRCLE

1810

1818

SIGNAL AT
FUNDAMENTAL
FREQUENCY
MOST
LIKELY VALID

NO

IS RELATIVE
PHASE OF HARMONIC
OUTSIDE OF GENERAL
POPULATION
RANGE?

1814

YES

1816

SIGNAL AT FUNDAMENTAL
FREQUENCY MOST LIKELY
INVALID (ALTHOUGH COULD
BE DUE TO ABNORMAL
PHYSIOLOGICAL CONDITIONS
SUCH AS MECHANICAL ASSIST)

FIG.18

**European Patent Office**

**EUROPEAN SEARCH REPORT**

Application Number

EP 05 25 3973

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 002 952 A (DIAB ET AL) 14 December 1999 (1999-12-14) <br><br> * figures 3,11-13 * <br> * column 12, line 47 - column 17, line 14 * | 1-9, 11-22, 24-26 | A61B5/145 |
| A | US 6 083 172 A (BAKER, JR. ET AL) 4 July 2000 (2000-07-04) <br> * column 20, line 47 - column 21, line 28 * | 9,22 | |
| A | US 2004/039273 A1 (TERRY ALVIN MARK) 26 February 2004 (2004-02-26) <br> * abstract * <br> * figures 1-11 * | 1,14 | |
| A | WO 01/25802 A (NTC TECHNOLOGY INC) 12 April 2001 (2001-04-12) <br> * figure 1 * <br> * page 12, lines 8-26 * <br> * page 15, line 6 - page 16, line 13 * | 1,14 | TECHNICAL FIELDS SEARCHED (Int.Cl.7) <br><br> A61B |
| A | US 4 911 167 A (CORENMAN ET AL) 27 March 1990 (1990-03-27) <br> * column 11, lines 5-36 * | 1,7,14, 20 | |

The present search report has been drawn up for all claims

| Place of search | Date of completion of the search | Examiner |
|---|---|---|
| Berlin | 29 September 2005 | Völlinger, M |

EPO FORM 1503 03.82 (P04C01)

**ANNEX TO THE EUROPEAN SEARCH REPORT
ON EUROPEAN PATENT APPLICATION NO.**                    EP 05 25 3973

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2005

| Patent document cited in search report | | Publication date | Patent family member(s) | | Publication date |
|---|---|---|---|---|---|
| US 6002952 | A | 14-12-1999 | AU<br>US<br>WO | 7109298 A<br>6067462 A<br>9846126 A1 | 11-11-1998<br>23-05-2000<br>22-10-1998 |
| US 6083172 | A | 04-07-2000 | NONE | | |
| US 2004039273 | A1 | 26-02-2004 | US<br>US | 2003163032 A1<br>2004171948 A1 | 28-08-2003<br>02-09-2004 |
| WO 0125802 | A | 12-04-2001 | AT<br>DE<br>EP | 284061 T<br>60016445 D1<br>1224566 A2 | 15-12-2004<br>05-01-2005<br>24-07-2002 |
| US 4911167 | A | 27-03-1990 | NONE | | |

For more details about this annex : see Official Journal of the European Patent Office, No. 12/82